(19) Europäisches Patentamt
European Patent Office
Office européen des brevets

(11) **EP 4 700 039 A1**

(12) **EUROPEAN PATENT APPLICATION**
published in accordance with Art. 153(4) EPC

(43) Date of publication:
**25.02.2026 Bulletin 2026/09**

(21) Application number: **24792090.3**

(22) Date of filing: **18.04.2024**

(51) International Patent Classification (IPC):
***C07K 7/06*** *(2006.01)* ***A61K 38/08*** *(2019.01)*
***A61P 17/00*** *(2006.01)* ***A61P 17/18*** *(2006.01)*
***A61P 17/16*** *(2006.01)*

(52) Cooperative Patent Classification (CPC):
Y02P 20/55

(86) International application number:
**PCT/CN2024/088620**

(87) International publication number:
**WO 2024/217505 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.04.2023 CN 202310428893**

(71) Applicant: **Winkey Technology USA Inc**
**Costa Mesa, California 92626 (US)**

(72) Inventors:
- **DING, Wenfeng**
  **Shenzhen, Guangdong 518000 (CN)**
- **XIAO, Yu**
  **Shenzhen, Guangdong 518000 (CN)**
- **ZHAO, Wenhao**
  **Shenzhen, Guangdong 518000 (CN)**
- **SUN, Xinlin**
  **Shenzhen, Guangdong 518000 (CN)**
- **GUAN, Fuyi**
  **Shenzhen, Guangdong 518000 (CN)**
- **CHEN, Xue**
  **Shenzhen, Guangdong 518000 (CN)**
- **PENG, Yan**
  **Shenzhen, Guangdong 518000 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) PEPTIDE, AND COMPOSITION THEREOF AND USE THEREOF

(57) Disclosed are a peptide having formula (I), i.e. $R_1$-$X_1$-$X_2$-$X_3$-Leu-Tyr-$X_4$-$X_5$-$R_2$, or a stereoisomer of the peptide, or a mixture of the stereoisomer of the peptide, or a salt of the peptide, or a mixture thereof; and the use thereof in the preparation of a composition for resisting aging or photoaging, anti-aging repair, soothing and moisturizing.

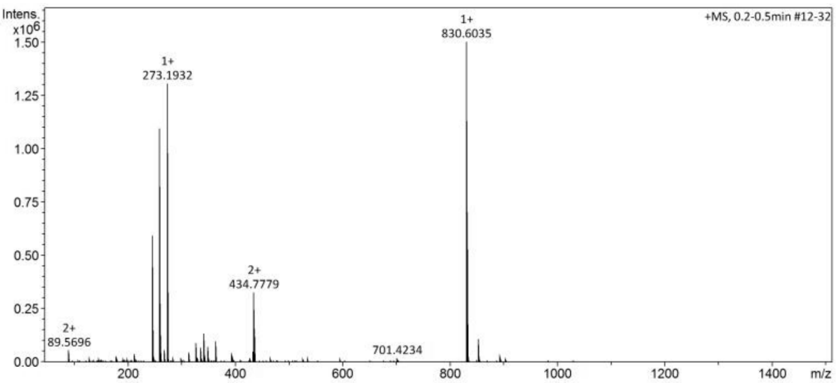


FIG. 1



EP 4 700 039 A1

## Description

The present application is based on the application with the CN application number 202310428893.9 and the filing date of April 20, 2023, and claims its priority. The disclosure of the CN application in its entirety is hereby incorporated into the present application.

## TECHNICAL FIELD

The present invention relates to the technical field of polypeptides, and particularly relates to a peptide capable of enhancing or repairing the skin barrier and a composition containing the same, and use thereof.

## BACKGROUND TECHNOLOGY

The skin is the first line of defense of the human body, and has functions such as protection, sensation, thermoregulation and metabolism. The skin barrier plays a key role in the skin's protective function. As an important line of defense for the skin, it can protect the skin from harm, defend against mechanical damage, prevent damage to tissues and organs, prevent the invasion of external harmful substances, resist oxidation, and provide protection against ultraviolet damage. It can also prevent the loss of various nutrients and water in the body, playing an important role in maintaining the stability of the human body's internal environment.

The skin barrier is composed of the sebum membrane, stratum corneum cells, and intercellular lipids. The stratum corneum cells are "piled up" within continuous intercellular lipids to form a special "brick-wall" structural system. The stratum corneum cells contain densely packed keratin, which forms many insoluble keratin fiber bundles. Among them, keratin is the main protein in stratum corneum cells. Only when it is expressed normally can the structural integrity of the skin barrier be maintained, thus playing its protective function for the skin. Filaggrin (FLG) is an important molecule that links keratin fibers in the stratum corneum of human skin. With the assistance of FLG monomer linkage, keratin fibers aggregate regularly to form a robust physical barrier at the outermost layer of the epidermis, thereby preventing transepidermal water loss and the invasion of external substances. FLG is distributed in different parts of the stratum corneum and is gradually degraded by enzymes into small-molecule substances, such as natural moisturizing factors, required by the stratum corneum during keratinocyte migration. It can be seen that filaggrin plays an important role in moisturizing and maintaining the integrity of the barrier.

In addition, hyaluronic acid is also an important component of the skin barrier, with its content in the skin varying with the processes of maturation and aging of skin. Hyaluronic acid can improve skin nutrition metabolism, lock in moisture on the skin surface, and make the skin soft and smooth. At the same time, it can protect cells from pathogen invasion, accelerate the recovery of skin tissue, improve the physiological properties of the skin, and has natural moisturizing and skin repair effects. With advancing age and under the influence of factors such as nutrition, sunlight exposure, and environmental conditions, the human body's ability to synthesize hyaluronic acid gradually declines, and the content of hyaluronic acid in the skin gradually decreases. When the hyaluronic acid content in the skin falls below a certain level, the water content in the skin's surface layer gradually decreases, leading to aging of stratum corneum cells and loss of water inside the skin, ultimately resulting in epidermal aging. Hyaluronic acid can also be decomposed by hyaluronidase into low-molecular-weight acidic irritants, thereby causing the release of histamine and inducing sensitive symptoms in the body.

Due to reasons such as environmental factors, dietary habits and improper skin care, more and more people are experiencing damaged skin barriers, which makes the skin's own defense capabilities insufficient, leading to issues such as skin sensitivity, aging, and dryness. Therefore, it is crucial to research solutions to the skin problems described above. At present, the main method to improve the skin barrier function is the use of moisturizers, including oil-blocking moisturizers, hydrophilic matrix moisture-absorbing moisturizers, hydrating moisturizers, and the like, but simple moisturizing cannot fundamentally solve the problem. Therefore, it is necessary to develop an active ingredient that can fundamentally resist aging and repair the skin by improving the skin barrier function, so as to meet people's growing demands for skin care.

## CONTENTS OF THE INVENTION

The object of the present invention is to provide a peptide capable of enhancing or repairing the skin barrier and a composition containing these peptides, as well as their uses in anti-aging or photoaging, anti-aging repair, soothing, moisturizing, etc.

In view of this, the present invention provides a peptide represented by formula (I), or a stereoisomer thereof, or a mixture of stereoisomers thereof, or a salt thereof,

$$R_1\text{-}X_1\text{-}X_2\text{-}X_3\text{-Leu-Tyr-}X_4\text{-}X_5\text{-}R_2 \qquad \text{(I)}$$

in formula (I),

$X_1$ is selected from -Gly-, -Lys- or a bond;

$X_2$ is selected from -Nle-, -Val-, -Leu- or -Ile-;

$X_3$ is selected from -Lys-, -Arg- or -His-;

$X_4$ is selected from -Gly- or a bond;

$X_5$ is selected from -Ser- or a bond;

$R_1$ is selected from H or $R_3$-CO-, wherein $R_3$ is selected from substituted or unsubstituted alkyl, substituted or unsubstituted alkenyl;

$R_2$ is selected from $-NR_4R_5$ or $-OR_4$, wherein each of $R_4$ and $R_5$ is independently selected from H, substituted or unsubstituted alkyl, substituted or unsubstituted alkenyl;

the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally selected from methyl, ethyl, isopropyl, isobutyl, tert-butyl, pentyl, hexyl, heptyl, octyl, decyl, dodecyl, tetradecyl, hexadecyl, octadecyl, 2-ethylhexyl, 2-methylbutyl, or 5-methylhexyl;

the alkenyl refers to a linear or branched chain alkenyl having 2-24 carbon atoms (optionally having 2-16 carbon atoms; optionally having 2-14 carbon atoms; optionally having 2-12 carbon atoms; optionally having 2, 3, 4, 5, or 6 carbon atoms); the alkenyl has one or more carbon-carbon double bonds, optionally has 1, 2 or 3 conjugated or non-conjugated carbon-carbon double bonds; the alkenyl is bonded to the rest portions of a molecule through a single bond; and is optionally selected from vinyl, oleyl, or linoleyl;

optionally, the substituent in the "substituted alkyl" or "substituted alkenyl" is selected from $C_1$-$C_4$ alkyl; hydroxyl; $C_1$-$C_4$ alkoxy; amino; $C_1$-$C_4$ aminoalkyl; $C_1$-$C_4$ carbonyloxy; $C_1$-$C_4$ oxycarbonyl; halogen (such as fluorine, chlorine, bromine, and iodine); cyano; nitro; azide; $C_1$-$C_4$ alkylsulfonyl; thiol; $C_1$-$C_4$ alkylthio; $C_6$-$C_{30}$ aryloxy such as phenoxy; $-NR_b(C{=}NR_b)NR_bR_c$, wherein $R_b$ and $R_c$ are independently selected from H, $C_1$-$C_4$ alkyl, $C_2$-$C_4$ alkenyl, $C_2$-$C_4$ alkynyl, $C_3$-$C_{10}$ cycloalkyl, $C_6$-$C_{18}$ aryl, $C_7$-$C_{17}$ aralkyl, a heterocyclic group having 3 to 10 members, or an amino protecting group.

Optionally, $R_1$ is selected from H, acetyl, tert-butyryl, hexanoyl, 2-methylhexanoyl, octanoyl, decanoyl, lauroyl, myristoyl, palmitoyl, stearoyl, oleoyl or linoleoyl; $R_4$ and $R_5$ are independently selected from H, methyl, ethyl, hexyl, dodecyl or hexadecyl;

optionally, $R_1$ is selected from H, acetyl, lauroyl, myristoyl or palmitoyl; $R_4$ is H and $R_5$ is selected from H, methyl, ethyl, hexyl, dodecyl or hexadecyl;

optionally, $R_1$ is H, acetyl or palmitoyl; $R_2$ is -OH or $-NH_2$.

Optionally, the peptide represented by formula (I), or a stereoisomer thereof, or a mixture of stereoisomers thereof, or a salt thereof, is selected from the following peptides (1)-(40):

(1) H-Gly-Nle-Lys-Leu-Tyr-OH;
(2) Ac-Gly-Nle-Lys-Leu-Tyr-$NH_2$;
(3) Palm-Gly-Nle-Lys-Leu-Tyr-OH;
(4) Palm-Gly-Nle-Lys-Leu-Tyr-$NH_2$;
(5) H-Val-Lys-Leu-Tyr-Gly-Ser-OH;
(6) H-Val-Lys-Leu-Tyr-Gly-Ser-$NH_2$;
(7) Ac-Val-Lys-Leu-Tyr-Gly-Ser-$NH_2$;
(8) Palm-Val-Lys-Leu-Tyr-Gly-Ser-OH;
(9) Palm-Val-Lys-Leu-Tyr-Gly-Ser-$NH_2$;

(10) H-Lys-Val-Lys-Leu-Tyr-OH;
(11) H-Lys-Val-Lys-Leu-Tyr-$NH_2$;
(12) Ac-Lys-Val-Lys-Leu-Tyr-$NH_2$;
(13) Palm-Lys-Val-Lys-Leu-Tyr-$NH_2$;
(14) Palm-Lys-Val-Lys-Leu-Tyr-OH;
(15) H-Gly-Nle-Arg-Leu-Tyr-OH;
(16) H-Gly-Nle-Arg-Leu-Tyr-$NH_2$;
(17) Ac-Gly-Nle-Arg-Leu-Tyr-OH;
(18) Ac-Gly-Nle-Arg-Leu-Tyr-$NH_2$;
(19) Palm-Gly-Nle-Arg-Leu-Tyr-OH;
(20) Palm-Gly-Nle-Arg-Leu-Tyr-$NH_2$;
(21) H-Lys-Leu-Lys-Leu-Tyr-OH;
(22) Ac-Lys-Leu-Lys-Leu-Tyr-$NH_2$;
(23) Ac-Gly-Ile-Lys-Leu-Tyr-$NH_2$;
(24) H-Gly-Leu-Arg-Leu-Tyr-Ser-OH;
(25) H-Gly-Val-Lys-Leu-Tyr-$NH_2$;
(26) Palm-Gly-Val-His-Leu-Tyr-$NH_2$;
(27) Ac-Gly-Nle-Arg-Leu-Tyr-Gly-OH;
(28) H-Nle-Lys-Leu-Tyr-Gly-OH;
(29) Ac-Ile-Lys-Leu-Tyr-Gly-Ser-$NH_2$;
(30) H-Lys-Nle-Arg-Leu-Tyr-OH;
(31) H-Ile-Lys-Leu-Tyr-Ser-$NH_2$;
(32) Ac-Gly-Nle-Lys-Leu-Tyr-OH;
(33) Palm-Gly-Leu-Lys-Leu-Tyr-$NH_2$;
(34) H-Gly-Leu-Lys-Leu-Tyr-Gly-OH;
(35) Ac-Ile-Lys-Leu-Tyr-Gly-$NH_2$;
(36) H-Ile-Arg-Leu-Tyr-Gly-Ser-OH;
(37) H-Val-His-Leu-Tyr-Gly-$NH_2$;
(38) Ac-Val-His-Leu-Tyr-Gly-Ser-OH;
(39) Palm-Gly-Leu-Arg-Leu-Tyr-Gly-$NH_2$;
(40) H-Gly-Leu-Arg-Leu-Tyr-Ser-OH.

Optionally, the peptide represented by formula (I), or a stereoisomer thereof, or a mixture of stereoisomers thereof, or a salt thereof, is selected from the following peptide (3), peptide (4), peptide (8), peptide (9), peptide (13), peptide (14), peptide (19), and peptide (20), specifically,

(3) Palm-Gly-Nle-Lys-Leu-Tyr-OH;

(4) Palm-Gly-Nle-Lys-Leu-Tyr-$NH_2$;

(8) Palm-Val-Lys-Leu-Tyr-Gly-Ser-OH;

(9) Palm-Val-Lys-Leu-Tyr-Gly-Ser-$NH_2$;

(13) Palm-Lys-Val-Lys-Leu-Tyr-$NH_2$;

(14) Palm-Lys-Val-Lys-Leu-Tyr-OH;

(19) Palm-Gly-Nle-Arg-Leu-Tyr-OH;

(20) Palm-Gly-Nle-Arg-Leu-Tyr-$NH_2$.

The peptides represented by formula (I) of the present invention can exist as stereoisomers or mixtures of stereoisomers; for example, the amino acids comprised therein can have the configuration L-, D-, or be racemic independently of each other. Thus, it is possible to obtain isomeric mixtures as well as racemic mixtures or diastereomeric mixtures, or pure diastereomers or enantiomers, depending on the number of asymmetric carbons and on which isomers or isomeric mixtures are present. The preferred structures of the peptides represented by formula (I) of the present invention are pure isomers, i.e., enantiomers or diastereomers.

For example, when -Lys- is mentioned in the present invention, it should be understood that -Lys- is selected from -L-Lys-, -D-Lys- or mixtures of both, racemic or non-racemic. The preparation methods described in this document enable the person skilled in the art to obtain each of the stereoisomers of the peptide of the present invention by choosing the amino acid with the right configuration.

The present invention further includes all suitable isotopic variants of the peptide represented by formula (1). These isotopic variants of the peptides of the present invention are herein understood as indicating compounds that: at least one atom in the peptides of the present invention is replaced with another atom of the same atomic number, but the atomic mass of the other atom is different from the atomic mass commonly or mainly present in nature. Examples of isotopes that can be incorporated into the peptides of the present invention are those of hydrogen, carbon, nitrogen, or oxygen, such as $^{2}H$ (deuterium), $^{3}H$ (tritium), $^{13}C$, $^{14}C$, $^{15}N$, $^{17}O$, or $^{18}O$. The specific isotopic variants of the peptides of the present invention (especially those into which one or more radioactive isotopes have been incorporated) may be advantageous, for example, in examining the mechanism of action or the distribution of active compounds in vivo; and due to their relatively simple preparability and detectability, compounds labeled with $^{3}H$ or $^{14}C$ isotopes are especially suitable for this purpose. In addition, due to the enhanced metabolic stability of compounds, the incorporation of isotopes (such as deuterium) can produce specific therapeutic benefits, such as prolonging the half-life in vivo or reducing the required active dosage. Therefore, in certain cases, such modifications of the peptides of the present application may also constitute preferred embodiments of the present invention. Isotopic variants of the peptides of the present invention can be prepared by methods known to those skilled in the art, such as the methods described further below and the methods described in the embodiments, using the respective reagents and/or corresponding isotopic modifications of starting substances.

The term "a salt" refers to a salt recognized for its use in animals, and more specifically in human beings, and includes a metal salt of the peptide represented by formula (I), wherein the metal includes, but is not limited to, lithium, sodium, potassium, calcium, magnesium, manganese, copper, zinc or aluminum, etc.; a salt formed from the peptide represented by formula (I) and an organic base, wherein the organic base includes, but is not limited to, ethylamine, diethylamine, ethylenediamine, ethanolamine, diethanolamine, arginine, lysine, histidine or piperazine among others; a salt formed from the peptide represented by formula (I) and an inorganic acid or an organic acid, wherein the organic acid includes, but is not limited to, acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid (pamoate) or gluconic acid, etc.; and the inorganic acid includes, but is not limited to, hydrochloric acid, sulfuric acid, boric acid or carbonic acid.

The synthesis of the peptide represented by formula (I) of the present invention or a stereoisomer thereof, or a salt thereof can be carried out according to conventional methods, known in the prior art, such as solid-phase synthesis methods, liquid-phase synthesis methods, or methods of combining solid-phase and liquid-phase, as well as by biotechnological methods with the aim of producing the desired sequences, or prepared by controlled hydrolysis of proteins with animal, fungal, or plant origins.

For example, a method of obtaining the peptides represented by formula (I) comprises the following steps:

- coupling of an amino acid with the N-terminal end protected and the C-terminal end free, with an amino acid with the N-terminal end free and the C-terminal end protected or bound to a solid carrier;
- elimination of the group protecting the N-terminal end;
- repetition of the coupling sequence and elimination of the group protecting the N-terminal end until the desired peptide sequence is obtained;
- elimination of the group protecting the C-terminal end or cleavage of the solid carrier.

Preferably, the C-terminal end is bound to a solid carrier and the method is carried out in solid phase including the coupling of an amino acid with the protected N-terminal end and the free C-terminal end with an amino acid with the N-terminal end free and the C-terminal end bound to a polymer carrier; elimination of the group protecting the N-terminal end; and repetition of this sequence as many times as is necessary to thus obtain the peptide of the desired length, followed by the cleavage of the synthesized peptide from the original polymeric carrier.

The functional groups of the side chains of the amino acids remain fully protected with temporary or permanent protecting groups throughout the synthesis, and can be deprotected simultaneously or orthogonally in the process of cleaving the peptide from the polymer carrier.

Alternatively, solid phase synthesis can be performed by a convergent strategy in which a dipeptide or tripeptide is coupled to a polymer support or to a dipeptide or amino acid previously bound to a polymer support.

The N-terminal and C-terminal ends are deprotected and/or the peptide is cleaved from the polymer support in an

indiscriminate order, using standard conditions and methods known in the art, and subsequently the terminal functional groups can be modified. Optional modifications to the N-terminal and C-terminal ends can be made to the peptide represented by formula (I) bound to the polymer support, or optional modifications to the N-terminal and C-terminal ends can be made after the peptide has been cleaved from the polymer support.

Another aspect of the present invention provides a composition, including an effective amount of the peptide represented by formula (I), or a stereoisomer thereof, or a mixture of stereoisomers thereof, or a salt thereof, and at least one excipient and optional an adjuvant.

Optionally, the adjuvant is selected from other agents of activating S1PR2 activity, analgesics, agents inhibiting PAR-2 activity, collagen synthesis stimulating agents, agents modulating PGC-1$\alpha$ synthesis, agents modulating PPAR$\gamma$ activity, agents increasing or reducing the triglyceride content of adipocytes, agents stimulating or delaying adipocyte differentiation, lipolytic agents or agents stimulating lipolysis, anti-cellulite agents, adipogenic agents, inhibitors of acetylcholine-receptor aggregation, agents inhibiting muscle contraction, anticholinergic agents, elastase inhibiting agents, matrix metalloproteinase inhibiting agents, melanin synthesis stimulating or inhibiting agents, whitening or depigmenting agents, propigmenting agents, self-tanning agents, antiaging agents, NO-synthase inhibiting agents, 5 $\alpha$ -reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin conditioners, substances that retain moisture, alpha hydroxy acids, beta hydroxy acids, moisturizers, hydrolytic epidermal enzymes, vitamins, amino acids, proteins, pigments, dyes, biopolymers, gelling polymers, thickening agents, surfactants, softening agents, binding agents, preservatives, anti-wrinkle agents, agents able to reduce or treat the bags under the eyes, keratolytic agents, antimicrobial agents, antifungal agents, fungistatic agents, bactericidal agents, bacteriostatic agents, agents stimulating the synthesis of dermal or epidermal macromolecules and/or capable of inhibiting or preventing their degradation, elastin synthesis-stimulation agents, decorin synthesis-stimulation agents, laminin synthesis-stimulation agents, defensin synthesis-stimulating agents, chaperone synthesis-stimulating agents, cAMP synthesis-stimulating agents, HSP70 synthesis stimulators, heat shock protein synthesis-stimulating agents, hyaluronic acid synthesis-stimulating agents, fibronectin synthesis-stimulating agents, sirtuin synthesis-stimulating agents, agents stimulating the synthesis of lipids and components of the stratum corneum, ceramides, fatty acids, agents that inhibit collagen degradation, agents that inhibit elastin degradation, agents that inhibit serine proteases, agents stimulating fibroblast proliferation, agents stimulating keratinocyte proliferation, agents stimulating adipocyte proliferation, agents stimulating melanocyte proliferation, agents stimulating keratinocyte differentiation, agents that inhibit acetylcholinesterase, skin relaxant agents, glycosaminoglycan synthesis-stimulating agents, antihyperkeratosis agents, comedolytic agents, anti-psoriasis agents, anti-eczema agents, DNA repair agents, DNA protecting agents, stabilizers, anti-itching agents, agents for the treatment and/or care of sensitive skin, firming agents, redensifying agents, restructuring agents, anti-stretch mark agents, agents regulating sebum production, antiperspirant agents, agents stimulating healing, coadjuvant healing agents, agents stimulating re-epithelization, coadjuvant re-epithelization agents, cytokines, calming agents, anti-inflammatory agents, anesthetic agents, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, perfumes, chelating agents, plant extracts, essential oils, marine extracts, agents obtained from a biofermentation process, mineral salts, cell extracts, sunscreens, and organic or mineral photoprotective agents active against ultraviolet A and/or B rays or mixtures thereof.

Optionally, a preparation of the composition is selected from creams, oils, balms, foams, lotions, gels, liniments, sera, ointments, mousses, powders, bars, pencils, sprays, aerosols, capsules, tablets, granules, chewing gum, solutions, suspensions, emulsions, elixirs, polysaccharide films, jellies or gelatins.

The peptides of the present invention have variable solubility in water according to the nature of their sequences or any potential modifications in the N-terminal and/or C-terminal ends. Therefore, the peptides of the present invention can be incorporated into the compositions via aqueous solution, and those that are not soluble in water can be solubilized in conventional solvents such as, but not limited to, ethanol, propanol, isopropanol, propylene glycol, glycerin, butylene glycol or polyethylene glycol or any combination thereof.

The effective amount of the peptide of the present invention to be administered, as well as doses thereof, will depend on many factors including age, the state of a user, the severity of a condition, the route and frequency of administration, and specific properties of the peptide to be used.

"An effective amount" refers to an amount of one or more peptides of the present invention that is non-toxic but sufficient to provide the desired effects. The peptide of the present invention is used in the composition of the present invention at an effective concentration to obtain the desired effects. In a preferred form, relative to the total weight of the composition, the concentration is between 0.00000001% (by weight) and 20% (by weight), preferably between 0.000001% (by weight) and 15% (by weight), more preferably between 0.0001% (by weight) and 10% (by weight), and even more preferably between 0.0001% (by weight) and 5% (by weight).

Another aspect of the present invention provides a delivery system or sustained release system, comprising an effective amount of the peptide represented by formula (I), or a stereoisomer thereof, or a mixture of stereoisomers thereof, or a salt thereof, or the aforementioned composition, to achieve better penetration of the active ingredient.

The term "delivery system" refers to a diluent, adjuvant, excipient or carrier administered with the peptide of the invention, selected from water, oil or surfactants, including those of petroleum, animal, plant, or synthetic origin, such as and not restricted to peanut oil, soybean oil, mineral oil, sesame oil, castor oil, polysorbates, sorbitan esters, ether sulfates, sulfates, betaines, glucosides, maltosides, fatty alcohols, nonoxynols, poloxamers, polyoxyethylenes, polyethylene glycols, dextrose, glycerin, digitonin and the like. A person skilled in the art knows the diluents which can be used in the different delivery systems in which the peptide of the invention can be administered.

The term "sustained release" is used in a conventional sense to refer to a delivery system that provides a gradual release of a compound over a period of time, and preferably, although not necessarily, with relatively constant compound release levels over the entire period of time.

Examples of the delivery system or sustained release system include liposomes, oleosomes, non-ionic surfactant liposome vesicles, ethosomes, millicapsules, microcapsules, nanocapsules, nanostructured lipid carriers, sponges, cyclodextrins, lipoid vesicles, micelles, millispheres, microspheres, nanospheres, lipospheres, microemulsions, nanoemulsions, milliparticles, microparticles or nanoparticles.

Another aspect of the present invention provides use of the peptide represented by formula (I), or a stereoisomer thereof, or a mixture of stereoisomers thereof, or a salt thereof, or the composition above, or the delivery system or sustained release system above in the preparation of a composition for anti-aging or photoaging.

Another aspect of the present invention provides use of the peptide represented by formula (I), or a stereoisomer thereof, or a mixture of stereoisomers thereof, or a salt thereof, or the composition above, or the delivery system or sustained release system above in the preparation of a composition for anti-aging repair, the anti-aging repair including one or more of improving cell activity, promoting cell proliferation and migration, enhancing or repairing the skin barrier.

Another aspect of the present invention provides use of the peptide represented by formula (I), or a stereoisomer thereof, or a mixture of stereoisomers thereof, or a salt thereof, or the composition above, or the delivery system or sustained release system above in the preparation of a composition for soothing.

Another aspect of the present invention provides use of the peptide represented by formula (I), or a stereoisomer thereof, or a mixture of stereoisomers thereof, or a salt thereof, or the composition above, or the delivery system or sustained release system above in the preparation of a composition for moisturizing.

In this description, the abbreviations used for amino acids follow the rules specified by the IUPAC-IUB Commission of Biochemical Nomenclature in the European Journal of Biochemistry (Eur. J. Biochem. 1984, 138:9-37).

Therefore, for example, Lys represents $NH_2$-$CH(CH_2CH_2CH_2CH_2NH_2)$-COOH, Lys-represents $NH_2$-$CH(CH_2CH_2CH_2CH_2NH_2)$-CO-, -Lys represents -NH-$CH(CH_2CH_2CH_2CH_2NH_2)$-COOH, and -Lys- represents -NH-$CH(CH_2CH_2CH_2CH_2NH_2)$-CO-. Thus, the hyphen, which represents the peptide bond, eliminates the OH in the 1-carboxyl group of the amino acid (represented here in the conventional non-ionized form) when located to the right of the symbol, and eliminates the H in the 2-amino group of the amino acid when located to the left of the symbol; both modifications can be applied to the same symbol (see Table 1).

Table 1 Structures of amino acid residues and one-letter and three-letter abbreviations thereof

| Name/Abbreviation | Residues | Name/Abbreviation | Residues |
|---|---|---|---|
| Glycyl -Gly-G | 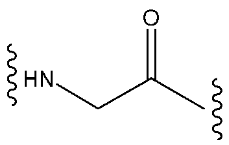 | Norleucyl -Nle- | 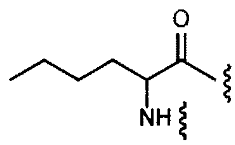 |
| Lysyl -Lys-K | 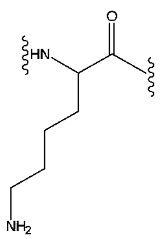 | Leucyl -Leu-L | 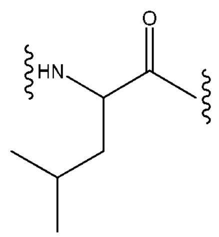 |

(continued)

| Name/Abbreviation | Residues | Name/Abbreviation | Residues |
|---|---|---|---|
| Tyrosyl -Tyr-Y | 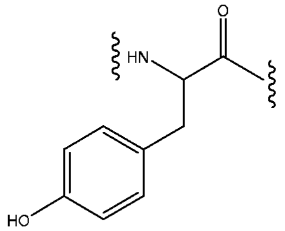 | Valyl -Val-V | 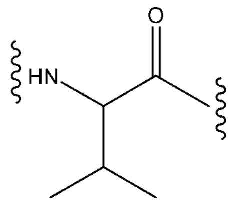 |
| Seryl -Ser-S | 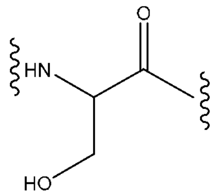 | Arginyl -Arg-R | 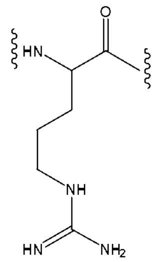 |

The abbreviation "Palm-" is used to represent palmitoyl in the present invention; Gly: glycine; Nle: norleucine; Lys: lysine; Leu: leucine; Tyr: tyrosine; Val: valine; Ser: serine; Arg: arginine.
The present invention has the following advantages and effects.

1. The peptide of the present invention is obtained through artificial design, easy to synthesize, safe and non-irritating to human body.

2. The peptide of the present invention can improve cell activity, promote cell proliferation and migration, enhance or repair the skin barrier, protect the skin from external damage, and has the effect of anti-aging repair; it also has the effect of anti-aging or photoaging.

3. The peptide of the present invention can increase the content of hyaluronic acid, promote the expression of filaggrin, and can also inhibit the activity of hyaluronidase, thereby enhancing or repairing the skin barrier and having moisturizing and soothing effects.

## DESCRIPTION OF THE DRAWINGS

To describe the technical solutions of the present invention more clearly, the following briefly describes the accompanying drawings required for describing the present invention. Apparently, the accompanying drawings in the following description show merely some embodiments of the present invention, and those of ordinary skill in the art may still obtain other accompanying drawings from these accompanying drawings without creative efforts.

FIG. 1 is a mass spectrum of the peptide (4) Palm-Gly-Nle-Lys-Leu-Tyr-$NH_2$ (molecular formula $C_{45}H_{79}N_7O_7$), in which the mass-to-charge ratio (m/z) of the $[M+H]^+$ quasimolecular ion peak is 830.6035, and the molecular weight measured by mass spectrometry is 829.60.

FIG. 2 is a mass spectrum of the peptide (9) Palm-Val-Lys-Leu-Tyr-Gly-Ser-$NH_2$ (molecular formula $C_{47}H_{82}N_8O_9$), in which the mass-to-charge ratio (m/z) of the $[M+H]^+$ quasimolecular ion peak is 903.6204, and the molecular weight measured by mass spectrometry is 902.62.

FIG. 3 is a mass spectrum of the peptide (13) Palm-Lys-Val-Lys-Leu-Tyr-$NH_2$ (molecular formula $C_{48}H_{86}N_8O_7$), in which the mass-to-charge ratio (m/z) of the $[M+H]^+$ quasimolecular ion peak is 887.6615, and the molecular weight measured by mass spectrometry is 886.66.

FIG. 4 is a mass spectrum of the peptide (14) Palm-Lys-Val-Lys-Leu-Tyr-OH (molecular formula $C_{48}H_{85}N_7O_8$), in which the mass-to-charge ratio (m/z) of the $[M+H]^+$ quasimolecular ion peak is 888.98, and the molecular weight measured by mass spectrometry is 887.98.

FIG. 5 is a mass spectrum of the peptide (20) Palm-Gly-Nle-Arg-Leu-Tyr-$NH_2$ (molecular formula $C_{45}H_{79}N_9O_7$), in which the mass-to-charge ratio (m/z) of the $[M+H]^+$ quasimolecular ion peak is 858.6092, and the molecular weight measured by mass spectrometry is 857.61.

FIG. 6 is a graph showing the effect results of test samples on the activity of HaCaT cells.

FIG. 7 is a graph showing the effect results of test samples on activity of photoaged cells.

FIG. 8 is a graph showing the results of cell scratch assay.

FIG. 9 is a graph showing the effect results of test samples on hyaluronic acid content.

FIG. 10 is a graph showing the effect results of test samples on the expression of FLG.

FIG. 11 is a graph showing the effect results of test samples on inhibition rate of hyaluronidase.

## SPECIFIC IMPLEMENTATIONS

To make the foregoing objects, features and advantages of the present invention more apparent and comprehensible, a detailed description is further made to the present invention below with reference to the accompanying drawings and the embodiments. Apparently, the described embodiments are only some embodiments rather than all the embodiments of the present invention. All other embodiments obtained by those of ordinary skill in the art based on the embodiments of the present invention without creative efforts shall fall within the protection scope of the present invention.

It should be noted that, in the present invention, the abbreviations used for amino acids follow the rules specified by the Commission of Biochemical Nomenclature of IUPAC-IUB in Eur. J. Biochem. (1984) 138: 9-37 and J. Chem. (1989) 264: 633-673.

Amide Resin: a starting resin for polypeptide synthesis (cross-linking degree 1%, substitution degree 1.72 mmol/g, grain size 100-200 mesh); Fmoc-Linker: 4-[(2,4-dimethoxyphenyl)(Fmoc-amino)methyl]phenoxyacetic acid; Wang Resin: 4-benzyloxybenzyl alcohol polystyrene; $Ac_2O$: acetic anhydride; DMF: N,N-dimethylformamide; DIPEA: diisopropylethylamine; DIC: diisopropylcarbodiimide; piperidine: piperidine; HOBt: 1-hydroxybenzotriazole; TFA: trifluoroacetic acid; TIS: triisopropylsilane; DMAP: 4-dimethylaminopyridine; Palm-OH: palmitic acid; Palm-: palmitoyl; Gly: glycine; Nle: norleucine; Lys: lysine; Leu: leucine; Tyr: tyrosine; Val: valine; Ser: serine; Arg: arginine; Fmoc: 9-fluorenylmethoxycarbonyl; Boc: tert-butoxycarbonyl; tBu: tert-butyl; Pbf: 2,2,4,6,7-pentamethyldihydrobenzofuran-5-sulfonyl.

EXAMPLE 1 Preparation of Palm-Gly-Nle-Lys-Leu-Tyr-$NH_2$

1.1 Preparation of Fmoc-Linker-Amide Resin

9 g of Amide Resin was weighed and added to a solid-phase synthesis reaction column, and swollen with DMF. The resin was washed and the solvent was removed.

8.7 g of Fmoc-Linker and 2.7 g of HOBt were weighed and added to a dry conical flask. After dissolving in DMF, the solution was cooled in an ice-water bath for 10 min, and 3.7 mL of DIC was added for activation for 10 min while avoiding water vapor.

The activated Fmoc-Linker was added to the swollen resin to react for 2.5 h, and then the reaction solution was removed. The resin was washed and the solvent was removed.

$Ac_2O$ and DIPEA were further added for capping for 1.5 h. The resin was washed and the solvent was removed.

1.2 Fmoc deprotection

Fmoc-Linker-Amide Resin was Fmoc-deprotected twice using 20% piperidine/DMF, each time for 10 min, and a sample was taken for the K-test, showing a dark blue color. The resin was washed with DMF 7 times, and the solvent was removed.

1.3 Feeding reaction

7.7 g of Fmoc-Tyr(tBu)-OH and 2.7 g of HOBt were weighed and added into a dry conical flask, the mixture was dissolved by adding DMF thereto, and the resultant was sealed and placed in a refrigerator at -18°C for 30 min. 3.9 mL of

DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 2.7 g of HOBt and 3.9 mL of DIC, 5.9 g of the activated Fmoc-Leu-OH was coupled to the peptidyl resin using DMF as a solvent, and the reaction was continued for 2 h. The resins were then washed and repeatedly subjected to the deprotection treatment of the Fmoc group so as to couple to the next amino acid. In each coupling, in the presence of 2.7 g of HOBt and 3.9 mL of DIC, 7.9 g of Fmoc-Lys(Boc)-OH, 7.1 g of Fmoc-Nle-OH and subsequent 5.9 g of Fmoc-Gly-OH were sequentially coupled using DMF as a solvent. After the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a dark blue color. The resin was washed with DMF 6 times, and the solvent was removed.

In the presence of 5.4 g of HOBt and 7.7 mL of DIC, 8.6 g of Palm-OH was coupled to the peptidyl resin using DMF as a solvent, the reaction was continued for 2.5 h, then the resin was washed, and the solvent was removed. After shrinkage drying, 19.4 g of Palm-Gly-Nle-Lys(Boc)-Leu-Tyr(tBu)-Linker-Amide Resin was obtained.

1.4 Cleavage

114 mL of TFA, 3 mL of TIS, and 3 mL of water were measured, mixed and stirred uniformly to obtain a cleavage solution, which was sealed and placed in a refrigerator at -18°C for subsequent use. Isopropyl ether was also placed in a -18°C refrigerator for subsequent use.

19.4 g of Palm-Gly-Nle-Lys(Boc)-Leu-Tyr(tBu)-Linker-Amide Resin was weighed and added to a round-bottom flask, the above-mentioned frozen cleavage solution was added, and the mixture was stirred and reacted for 2 h. The mixture was filtered and the filtrate was collected and concentrated to 15 mL, then isopropyl ether was added, stirred, centrifuged and washed 6 times, and the resultant was vacuum dried to obtain 14.0 g of crude Palm-Gly-Nle-Lys-Leu-Tyr-$NH_2$ peptide.

1.5 Purification

14.0 g of crude peptide was weighed and dissolved in 360 mL of mixed solution of acetic acid, methanol and water, and the resultant was filtered through a 0.22 μm microporous filter membrane to afford a clear and transparent solution. The solution was subjected to reversed-phase HPLC purification, and the purification gradient is shown in the following table:

| Time (min) | Flow rate (mL/min) | A% (acetonitrile) | B% (0.1% acetic acid + pure water) |
|---|---|---|---|
| 0 | 40 | 25 | 75 |
| 10 | 40 | 25 | 75 |
| 20 | 40 | 50 | 50 |
| 30 | 40 | 65 | 35 |
| 60 | 40 | 65 | 35 |

The filtered sample was injected for purification. Fractions were collected, concentrated and freeze-dried to afford the peptide (4) Palm-Gly-Nle-Lys-Leu-Tyr-$NH_2$ with a purity of 97.16%.

EXAMPLE 2 Preparation of Palm-Val-Lys-Leu-Tyr-Gly-Ser-$NH_2$

2.1 Preparation of Fmoc-Linker-Amide Resin

9 g of Amide Resin was weighed and added to a solid-phase synthesis reaction column, and swollen with DMF. The resin was washed and the solvent was removed.

8.7 g of Fmoc-Linker and 2.6 g of HOBt were weighed and added to a dry conical flask. After dissolving in DMF, the solution was cooled in an ice-water bath for 10 min, and 3.7 mL of DIC was added for activation for 10 min while avoiding water vapor.

The activated Fmoc-Linker was added to the swollen resin to react for 2.5 h, and then the reaction solution was removed. The resin was washed and the solvent was removed.

$Ac_2O$ and DIPEA were further added for capping for 1.5 h. The resin was washed and the solvent was removed.

2.2 Fmoc deprotection

Fmoc-Linker-Amide Resin was Fmoc-deprotected twice using 20% piperidine/DMF, each time for 10 min, and a sample was taken for the K-test, showing a dark blue color. The resin was washed with DMF 7 times, and the solvent was removed.

2.3 Feeding reaction

6.5 g of Fmoc-Ser(tBu)-OH and 2.7 g of HOBt were weighed and added into a dry conical flask, the mixture was dissolved by adding DMF thereto, and the resultant was sealed and placed in a refrigerator at -18°C for 30 min. 3.9 mL of DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 2.7 g of HOBt and 3.7 mL of DIC, 5.0 g of the activated Fmoc-Gly-OH was coupled to the peptidyl resin using DMF as a solvent, and the reaction was continued for 2 h. The resins were then washed and repeatedly subjected to the deprotection treatment of the Fmoc group so as to couple to the next amino acid. In each coupling, in the presence of 2.7 g of HOBt and 3.7 mL of DIC, 7.7 g of Fmoc-Tyr(tBu)-OH, 7.1 g of Fmoc-Leu-OH, 9.4 g of Fmoc-Lys(Boc)-OH and subsequent 6.8 g of Fmoc-Val-OH were sequentially coupled using DMF as a solvent. After the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a dark blue color. The resin was washed with DMF 6 times, and the solvent was removed.

In the presence of 5.4 g of HOBt and 7.7 mL of DIC, 8.6 g of Palm-OH was coupled to the peptidyl resin using DMF as a solvent, the reaction was continued for 2.5 h, then the resin was washed, and the solvent was removed. After shrinkage drying, 21.5 g of Palm-Val-Lys(Boc)-Leu-Tyr(tBu)-Gly-Ser(tBu)-Linker-Amide Resin was obtained.

2.4 Cleavage

114 mL of TFA, 3 mL of TIS, and 3 mL of water were measured, mixed and stirred uniformly to obtain a cleavage solution, which was sealed and placed in a refrigerator at -18°C for subsequent use. Isopropyl ether was also placed in a -18°C refrigerator for subsequent use.

21.5 g of Palm-Val-Lys(Boc)-Leu-Tyr(tBu)-Gly-Ser(tBu)-Linker-Amide Resin was weighed and added to a round-bottom flask, the above-mentioned frozen cleavage solution was added, and the mixture was stirred and reacted for 2 h. The mixture was filtered and the filtrate was collected and concentrated to 15 mL, then isopropyl ether was added, stirred, centrifuged and washed 6 times, and the resultant was vacuum dried to obtain 10.3 g of crude Palm-Val-Lys-Leu-Tyr-Gly-Ser-$NH_2$ peptide.

2.5 Purification

10.3 g of crude peptide was weighed and dissolved in 800 mL of mixed solution of acetic acid and water, and the resultant was filtered through a 0.22 $\mu$m microporous filter membrane to afford a clear and transparent solution. The solution was subjected to reversed-phase HPLC purification, and the purification gradient is shown in the following table:

| Time (min) | Flow rate (mL/min) | A% (acetonitrile) | B% (0.1% acetic acid + pure water) |
|---|---|---|---|
| 0 | 40 | 10 | 90 |
| 10 | 40 | 25 | 75 |
| 20 | 40 | 50 | 50 |
| 30 | 40 | 65 | 35 |
| 60 | 40 | 65 | 35 |

The filtered sample was injected for purification. Fractions were collected, concentrated and freeze-dried to afford the peptide (9) Palm-Val-Lys-Leu-Tyr-Gly-Ser-$NH_2$ with a purity of 97.08%.

EXAMPLE 3 Preparation of Palm-Lys-Val-Lys-Leu-Tyr-$NH_2$

3.1 Preparation of Fmoc-Linker-Amide Resin

9 g of Amide Resin was weighed and added to a solid-phase synthesis reaction column, and swollen with DMF. The resin was washed and the solvent was removed.

8.7 g of Fmoc-Linker and 2.6 g of HOBt were weighed and added to a dry conical flask. After dissolving in DMF, the solution was cooled in an ice-water bath for 10 min, and 3.7 mL of DIC was added for activation for 10 min while avoiding water vapor.

The activated Fmoc-Linker was added to the swollen resin to react for 2.5 h, and then the reaction solution was removed. The resin was washed and the solvent was removed.

$Ac_2O$ and DIPEA were further added for capping for 1.5 h. The resin was washed and the solvent was removed.

3.2 Fmoc deprotection

Fmoc-Linker-Amide Resin was Fmoc-deprotected twice using 20% piperidine/DMF, each time for 10 min, and a sample was taken for the K-test, showing a dark blue color. The resin was washed with DMF 7 times, and the solvent was removed.

3.3 Feeding reaction

7.7 g of Fmoc-Tyr(tBu)-OH and 2.7 g of HOBt were weighed and added into a dry conical flask, the mixture was dissolved by adding DMF thereto, and the resultant was sealed and placed in a refrigerator at -18°C for 30 min. 3.9 mL of DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 2.7 g of HOBt and 3.9 mL of DIC, 5.9 g of the activated Fmoc-Leu-OH was coupled to the peptidyl resin using DMF as a solvent, and the reaction was continued for 2 h. The resins were then washed and repeatedly subjected to the deprotection treatment of the Fmoc group so as to couple to the next amino acid. In each coupling, in the presence of 2.7 g of HOBt and 3.9 mL of DIC, 7.9 g of Fmoc-Lys(Boc)-OH, 6.8 g of Fmoc-Val-OH and subsequent 9.4 g of Fmoc-Lys(Boc)-OH were sequentially coupled using DMF as a solvent. After the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a dark blue color. The resin was washed with DMF 6 times, and the solvent was removed.

In the presence of 5.4 g of HOBt and 7.7 mL of DIC, 8.6 g of Palm-OH was coupled to the peptidyl resin using DMF as a solvent, the reaction was continued for 2.5 h, then the resin was washed, and the solvent was removed. After shrinkage drying, 19.8 g of Palm-Lys(Boc)-Val-Lys(Boc)-Leu-Tyr(tBu)-Linker-Amide Resin was obtained.

3.4 Cleavage

152 mL of TFA, 4 mL of TIS, and 4 mL of water were measured, mixed and stirred uniformly to obtain a cleavage solution, which was sealed and placed in a refrigerator at -18°C for subsequent use. Isopropyl ether was also placed in a -18°C refrigerator for subsequent use.

19.8 g of Palm-Lys(Boc)-Val-Lys(Boc)-Leu-Tyr(tBu)-Linker-Amide Resin was weighed and added to a round-bottom flask, the above-mentioned frozen cleavage solution was added, and the mixture was stirred and reacted for 2 h. The mixture was filtered and the filtrate was collected and concentrated to 15 mL, then isopropyl ether was added, stirred, centrifuged and washed 6 times, and the resultant was vacuum dried to obtain 6.3 g of crude Palm-Lys-Val-Lys-Leu-Tyr-$NH_2$ peptide.

3.5 Purification

6.3 g of crude peptide was weighed and dissolved in 450 mL of mixed solution of acetic acid, methanol and water, and the resultant was filtered through a 0.22 $\mu$m microporous filter membrane to afford a clear and transparent solution. The solution was subjected to reversed-phase HPLC purification, and the purification gradient is shown in the following table:

| Time (min) | Flow rate (mL/min) | A% (acetonitrile) | B% (0.1% acetic acid + pure water) |
|---|---|---|---|
| 0 | 40 | 10 | 90 |
| 10 | 40 | 25 | 75 |
| 20 | 40 | 35 | 65 |
| 30 | 40 | 40 | 60 |
| 50 | 40 | 50 | 50 |
| 80 | 40 | 60 | 40 |

The filtered sample was injected for purification. Fractions were collected, concentrated and freeze-dried to afford the peptide (13) Palm-Lys-Val-Lys-Leu-Tyr-$NH_2$ with a purity of 96.55%.

EXAMPLE 4 Preparation of Palm-Lys-Val-Lys-Leu-Tyr-OH

4.1 Swelling and reaction of resin

10 g of Wang Resin was weighed and added to a solid-phase synthesis reaction column, and swollen with DMF. The resin was washed and the solvent was removed.

11.0 g of Fmoc-Tyr(tBu)-OH and 4.0 g of HOBt were weighed and added to a dry conical flask. After dissolving in DMF, the solution was cooled in an ice-water bath for 10 min, and 6 mL of DIC was added for activation for 10 min while avoiding water vapor.

The activated Fmoc-Tyr(tBu)-OH and 1.5 g of DMAP were added to the swollen resin to react for 2.5 h, and then the reaction solution was removed. The resin was washed and the solvent was removed.

$Ac_2O$, DIPEA and DMAP were further added for capping for 1.5 h. The resin was washed and the solvent was removed.

4.2 Fmoc deprotection

Fmoc-Tyr(tBu)-Wang Resin was Fmoc-deprotected twice using 20% piperidine/DMF, each time for 10 min, and a sample was taken for the K-test, showing a dark blue color. The resin was washed with DMF 7 times, and the solvent was removed.

4.3 Feeding reaction

7 g of Fmoc-Leu-OH and 3 g of HOBt were weighed and added to a dry conical flask, and the resultant was dissolved by adding DMF and then sealed and placed in a refrigerator at -18°C for 30 min. 5 mL of DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 3 g of HOBt and 5 mL of DIC, 9 g of the activated Fmoc-Lys(Boc)-OH was coupled to the peptidyl resin using DMF as a solvent, and the reaction was continued for 2 h. The resins were then washed and repeatedly subjected to the deprotection treatment of the Fmoc group so as to couple to the next amino acid. In each coupling, in the presence of 3 g of HOBt and 5 mL of DIC, 8 g of Fmoc-Val-OH and subsequent 11 g of Fmoc-Lys(Boc)-OH were sequentially coupled using DMF as a solvent. After the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a dark blue color. The resin was washed with DMF 6 times, and the solvent was removed.

In the presence of 7 g of HOBt and 9 mL of DIC, 10 g of Palm-OH was coupled to the peptidyl resin using DMF as a solvent, the reaction was continued for 2.5 h, then the resin was washed, and the solvent was removed. After shrinkage drying, 23 g of Palm-Lys(Boc)-Val-Lys(Boc)-Leu-Tyr(tBu)-Wang Resin was obtained.

4.4 Cleavage

131 mL of TFA, 3.5 mL of TIS, and 3.5 mL of water were measured, mixed and stirred uniformly to obtain a

cleavage solution, which was sealed and placed in a refrigerator at -18°C for subsequent use. Isopropyl ether was also placed in a -18°C refrigerator for subsequent use.

23 g of Palm-Lys(Boc)-Val-Lys(Boc)-Leu-Tyr(tBu)-Wang Resin was weighed and added to a round-bottom flask, the above-mentioned frozen cleavage solution was added, and the mixture was stirred and reacted for 2 h. The mixture was filtered and the filtrate was collected and concentrated to 15 mL, then isopropyl ether was added, stirred, centrifuged and washed 6 times, and the resultant was vacuum dried to obtain 13 g of crude Palm-Lys-Val-Lys-Leu-Tyr-OH peptide.

4.5 Purification

13 g of crude peptide was weighed and dissolved in 220 mL of mixed solution of acetic acid, methanol and water, and the resultant was filtered through a 0.22 μm microporous filter membrane to afford a clear and transparent solution. The solution was subjected to reversed-phase HPLC purification, and the purification gradient is shown in the following table:

| Time (min) | Flow rate (mL/min) | A% (acetonitrile) | B% (0.1% acetic acid + pure water) |
|---|---|---|---|
| 0 | 40 | 25 | 75 |
| 15 | 40 | 35 | 65 |
| 30 | 40 | 45 | 55 |
| 45 | 40 | 60 | 40 |
| 80 | 40 | 65 | 35 |
| 100 | 40 | 65 | 35 |

The filtered sample was injected for purification. Fractions were collected, concentrated and freeze-dried to afford the peptide (14) Palm-Lys-Val-Lys-Leu-Tyr-OH with a purity of 97.615%.

EXAMPLE 5 Preparation of Palm-Gly-Nle-Arg-Leu-Tyr-$NH_2$

5.1 Preparation of Fmoc-Linker-Amide Resin

9 g of Amide Resin was weighed and added to a solid-phase synthesis reaction column, and swollen with DMF. The resin was washed and the solvent was removed.

8.7 g of Fmoc-Linker and 2.6 g of HOBt were weighed and added to a dry conical flask. After dissolving in DMF, the solution was cooled in an ice-water bath for 10 min, and 3.7 mL of DIC was added for activation for 10 min while avoiding water vapor.

The activated Fmoc-Linker was added to the swollen resin to react for 2.5 h, and then the reaction solution was removed. The resin was washed and the solvent was removed.

$Ac_2O$ and DIPEA were further added for capping for 1.5 h. The resin was washed and the solvent was removed.

5.2 Fmoc deprotection

Fmoc-Linker-Amide Resin was Fmoc-deprotected twice using 20% piperidine/DMF, each time for 10 min, and a sample was taken for the K-test, showing a dark blue color. The resin was washed with DMF 7 times, and the solvent was removed.

5.3 Feeding reaction

7.7 g of Fmoc-Tyr(tBu)-OH and 2.7 g of HOBt were weighed and added to a dry conical flask, the mixture was dissolved by adding DMF thereto, and the resultant was sealed and placed in a refrigerator at -18°C for 30 min. 3.9 mL of DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 2.7 g of HOBt and 3.9 mL of DIC, 5.9 g of the activated Fmoc-Leu-OH was coupled to the peptidyl resin using DMF as a solvent, and the reaction was continued for 2 h. The resins were then washed and repeatedly subjected to the deprotection treatment of the Fmoc group so as to couple to

the next amino acid. In each coupling, in the presence of 2.7 g of HOBt and 3.9 mL of DIC, 15.2 g of Fmoc-Arg(Pbf)-OH, 7.1 g of Fmoc-Nle-OH and subsequent 5.9 g of Fmoc-Gly-OH were sequentially coupled using DMF as a solvent. After the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a dark blue color. The resin was washed with DMF 6 times, and the solvent was removed.

In the presence of 5.4 g of HOBt and 7.7 mL of DIC, 8.6 g of Palm-OH was coupled to the peptidyl resin using DMF as a solvent, the reaction was continued for 1.5 h, then the resin was washed, and the solvent was removed. After shrinkage drying, 18.2 g of Palm-Gly-Nle-Arg(Pbf)-Leu-Tyr(tBu)-Linker-Amide Resin was obtained.

5.4 Cleavage

152 mL of TFA, 4 mL of TIS, and 4 mL of water were measured, mixed and stirred uniformly to obtain a cleavage solution, which was sealed and placed in a refrigerator at -18°C for subsequent use. Isopropyl ether was also placed in a -18°C refrigerator for subsequent use.

18.2 g of Palm-Gly-Nle-Arg(Pbf)-Leu-Tyr(tBu)-Linker-Amide Resin was weighed and added to a round-bottom flask, the above-mentioned frozen cleavage solution was added, and the mixture was stirred and reacted for 2 h. The mixture was filtered and the filtrate was collected and concentrated to 15 mL, then isopropyl ether was added, stirred, centrifuged and washed 6 times, and the resultant was vacuum dried to obtain 13 g of crude Palm-Gly-Nle-Arg-Leu-Tyr-$NH_2$ peptide.

5.5 Purification

13 g of crude peptide was weighed and dissolved in 220 mL of mixed solution of acetic acid and water, and the resultant was filtered through a 0.22 μm microporous filter membrane to afford a clear and transparent solution. The solution was subjected to reversed-phase HPLC purification, and the purification gradient is shown in the following table:

| Time (min) | Flow rate (mL/min) | A% (acetonitrile) | B% (0.1% acetic acid + pure water) |
|---|---|---|---|
| 0 | 40 | 25 | 75 |
| 10 | 40 | 25 | 75 |
| 20 | 40 | 55 | 45 |
| 30 | 40 | 65 | 35 |
| 50 | 40 | 70 | 30 |
| 80 | 40 | 70 | 30 |

The filtered sample was injected for purification. Fractions were collected, concentrated and freeze-dried to afford the peptide (20) Palm-Gly-Nle-Arg-Leu-Tyr-$NH_2$ with a purity of 98.23%.

EXAMPLE 6

Other peptides of formula (I) of the present invention can be prepared by similar methods.

The molecular weight of the obtained peptides was determined by ESI-MS. The test results of some peptides are shown in Table 2 below and FIGS. 1-5.

Table 2 Results of determination of molecular weight by mass spectrometry

| Serial number | Sequence | Molecular weight mass spectrometry analysis results |
|---|---|---|
| (4) | Palm-Gly-Nle-Lys-Leu-Tyr-$NH_2$ | 829.60 |
| (9) | Palm-Val-Lys-Leu-Tyr-Gly-Ser-$NH_2$ | 902.62 |
| (13) | Palm-Lys-Val-Lys-Leu-Tyr-$NH_2$ | 886.66 |
| (14) | Palm-Lys-Val-Lys-Leu-Tyr-OH | 887.98 |
| (20) | Palm-Gly-Nle-Arg-Leu-Tyr-$NH_2$ | 857.61 |

EXAMPLE 7 Cell proliferation assay

7.1 Reagents and materials

Thiazolyl blue (MTT), dimethyl sulfoxide (DMSO), DMEM culture medium, fetal bovine serum, and PBS.

7.2 Instruments

A microplate reader, a $CO_2$ incubator, and an ultra-clean workbench.

7.3 Cell lines

Human keratinocytes (HaCaT) were purchased from the Kunming Cell Bank of China Center for Type Culture Collection under the Chinese Academy of Sciences.

7.4 Samples to be tested

Polypeptide group: Peptide (4), peptide (9), peptide (13) and peptide (20). The test concentrations of all the above samples were 6.25 ppm, 12.5 ppm and 25 ppm.

Blank control group: PBS.

Positive control group: 2% DMSO.

7.5 Experimental protocol

Cells in good exponential growth phase were taken. A 0.25% trypsin digestion solution was added so that digestion allowed the adherent cells to fall off, counting 1 to 4 $\times 10^5$ cells/mL, which were prepared into a cell suspension.
The cell suspension was inoculated into a 96-well plate with 200 μL/well, and cultured in a constant-temperature $CO_2$ incubator for 24 h.
After changing the medium, samples of the polypeptide groups, the blank control group and the positive control group were added at 20 μ L/well respectively, and incubated in a 5% $CO_2$ incubator at 37°C for 72 h.
20 μL of 5 mg/mL MTT was added to each well and the incubation was continued in the 5% $CO_2$ incubator at 37°C for 4 h. The original solution was removed and 150 μL/well of DMSO was added. After shaking the plate on a plate shaker for 5 min, a microplate reader was used to determine the OD value of each well at a wavelength of 570 nm, and the cell viability was calculated.

Cell viability=[OD (test sample well)-OD (zero adjustment well)]/[OD (blank control well)-OD (zero adjustment well)] $\times$ 100%

7.6 Experimental results

The MTT assay is a method of detecting cell survival and growth. The measured OD values are directly proportional to cell viability. The higher the cell viability, the larger the OD value. Conversely, when the cell viability is significantly less than 80%, it is considered to exhibit significant cytotoxicity.
The effect results of the test samples on HaCaT cell viability are shown in FIG. 6. The results showed that compared with the blank control group, the HaCaT cell viability in the positive control group decreased significantly, indicating that 2% DMSO had a toxic effect on HaCaT cells, while the polypeptide groups all had no toxic effect. Among them, peptide (9) and peptide (20) could significantly improve cell activity and promote the proliferation of HaCaT keratinocytes at low concentrations.
From this, it can be known that the peptide of the present invention can improve the activity of HaCaT keratinocytes, promote the proliferation of HaCaT keratinocytes, thereby repairing the skin barrier and protecting the skin from external damage.

EXAMPLE 8 Photoaging experiment

8.1 Reagents and materials

Fetal bovine serum, DMEM culture medium, PBS, penicillin, streptomycin, and thiazolyl blue (MTT).

8.2 Instruments

A microplate reader, a $CO_2$ incubator, and an ultra-clean workbench.

8.3 Cell lines

Human keratinocytes (HaCaT) were purchased from the Kunming Cell Bank of China Center for Type Culture Collection under the Chinese Academy of Sciences.

8.4 Samples to be tested

Polypeptide group: Peptide (13) and peptide (20). The test concentrations of all the above samples were 6.25 ppm and 12.5 ppm.
Blank control group: PBS.
UV group: UV radiation with PBS.

8.5 Experimental protocol

Cells in good exponential growth phase were taken. A 0.25% trypsin digestion solution was added so that digestion allowed the adherent cells to fall off, counting 1 to $4 \times 10^5$ cells/mL, which were prepared into a cell suspension.
The cell suspension was properly diluted and inoculated into a 96-well plate at 10,000 cells/well. When the cells reached approximately 80% confluence, an UV photoaging model was established. In the blank control group, 50 μL of PBS was added, and supplemented with the culture medium to 200 μL, while no UV irradiation was performed. For the UV group and the polypeptide groups, a proper amount of PBS was added to wash the culture repeatedly until colorless, followed by adding 50 μL of PBS and irradiating under an 80 mJ/cm$^2$ UV light. The distance between the light source and the culture bottle was 15 cm. After irradiation, the PBS was removed. In the UV group, a PBS solution and the culture medium were added to 200 μL; in the polypeptide groups, the culture medium and the samples diluted in a certain ratio were added to 200 μL. The blank control group, the UV group, and the polypeptide groups were further incubated in a 5% $CO_2$ incubator at 37°C for 24 h.
Then 22 μL of 5 mg/mL MTT was added to each well and the incubation was continued in the 5% $CO_2$ incubator at 37°C for 4 h. The original solution was removed and 150 μL/well of DMSO was added. After shaking the plate on a plate shaker for 5 min, a microplate reader was used to read the reference OD values at wavelengths of 490 nm and 630 nm.

8.6 Experimental results

Skin aging is affected by endogenous and exogenous factors, such as genetics, environmental exposure, UV irradiation, hormonal changes, etc. The accumulation of these factors, especially exposure to UV irradiation, leads to changes in structure, function and appearance of the skin. In this experiment, 80 mJ/cm$^2$ UV energy was selected for radiation to establish a skin photoaging model.
The effect results of the test samples on photoaged cell activity are shown in FIG. 7. Compared with the blank control group, the HaCaT cell activity in UV group was significantly decreased after UV radiation, indicating that the photoaging model was successfully established. Compared with the UV group, the polypeptide group could significantly increase cell activity at low concentrations, thereby improving cellular senescence and producing significant anti-photoaging effect.
From this, it can be known that the peptide of the present invention has an excellent anti-photoaging effect and can be used to delay aging.

EXAMPLE 9 Cell scratch assay

9.1 Reagents and materials

Fetal bovine serum, DMEM culture medium, PBS, penicillin, and streptomycin.

9.2 Instruments

An optical microscope, and a $CO_2$ incubator.

9.3 Cell lines

Human keratinocytes (HaCaT) were purchased from the Kunming Cell Bank of China Center for Type Culture Collection under the Chinese Academy of Sciences.

9.4 Samples to be tested

Polypeptide group: Peptide (13) and peptide (20). The test concentrations of all the above samples were 12.5 ppm.

Blank control group: PBS.

Positive control group: 50 U/mL of EGF.

9.5 Experimental protocol

A bottle of HaCaT cells in good exponential growth phase was taken. A 0.25% trypsin digestion solution was added so that digestion allowed the adherent cells to fall off, counting 1 to $4 \times 10^5$ cells/mL, which were prepared into a cell suspension.

The cells were inoculated into a 24-well plate at a density of 150,000 cells/well, with 2 mL cell suspension per well. After the cells attached to the wall and grew to 100% confluence, the culture medium was replaced with a medium containing 0.5% - 1% fetal bovine serum to maintain the cells for 24 h for synchronization. A sterilized pipette tip was used to scratch a wound, the detached cells were washed off with PBS, and the samples to be tested were added. The incubation was continued in the 5% $CO_2$ incubator at 37°C, and the migration of cells was observed under an optical microscope after 24 h.

9.6 Experimental results

The scratch migration experiment of cells can reflect the proliferation and repair ability of cells. A scratch assay was performed on the basis of HaCaT cells growing all over the dish, and the migration of cells was observed under an optical microscope 24 h after administration of the samples to evaluate the effect of the test samples on cell proliferation and migration.

The results of the scratch experiment of cells are shown in FIG. 8. The results showed that the scratches of cells in the blank control group were still obvious and the scratch spacing was normal. Compared with the blank control group, the cells in the EGF positive control group showed obvious cell proliferation and migration, indicating that the modeling was successful. The scratch spacings of the polypeptide groups were shorter than that of the blank control group, and cell migration tracks appeared.

It can be seen from this that the peptide of the present invention can promote cell proliferation and migration and have skin repair effects.

EXAMPLE 10 Determination of hyaluronic acid content

10.1 Reagents and materials

Fetal bovine serum, DMEM culture medium, PBS, trypsin, hyaluronic acid detection kit, RIPA lysis buffer and BCA protein kit.

10.2 Instruments

A microplate reader, a $CO_2$ incubator, an ultra-clean workbench, and a thermostat.

10.3 Cell lines

Human keratinocytes (HaCaT) were purchased from the Kunming Cell Bank of China Center for Type Culture Collection under the Chinese Academy of Sciences.

10.4 Samples to be tested

Polypeptide group: Peptide (4), peptide (13) and peptide (20). The test concentrations of all the above samples were 12.5 ppm.
Blank control group: PBS.

10.5 Experimental protocol

A bottle of HaCaT cells in good exponential growth phase was taken. A 0.25% trypsin digestion solution was added so that digestion allowed the adherent cells to fall off, counting 1 to $4 \times 10^6$ cells/mL, which were prepared into a cell suspension.
The cells were inoculated into a 6-well plate at $10^5$ cells/well, and grouped when the cells reached approximately 80% confluence. The blank control group was added with 200 μL of PBS, and supplemented with the culture medium to 800 μL. The polypeptide groups were added with 200 μL of samples, and supplemented with the culture medium to 800 μL. The blank control group and the polypeptide groups were incubated in a 5% $CO_2$ incubator at 37°C for 48 h.
After completion of the culture, cells were scraped off using a cell scraper, and 200 μL of RIPA lysis buffer was added to extract proteins. The mixture was incubated on ice for 15 min, and centrifuged at 10,000 g for 10 min to collect the supernatant. The total protein concentration was quantified to 1 mg/mL using the BCA method. The procedure was performed according to the operation instructions of the hyaluronic acid detection kit. The OD value of each well was measured in sequence at 570 nm using a microplate reader within 15 min.

10.6 Experiment results

Hyaluronic acid is one of the main matrix ingredients of the human skin epidermis and an important component of the skin barrier. It can lock in moisture on the skin surface, protect cells from pathogen invasion, accelerate the recovery of skin tissue, improve the physiological properties of the skin, and has natural moisturizing and skin repair effects. The reduction in hyaluronic acid content or its destruction in the skin can cause moisture loss and epidermal aging, so hyaluronic acid is also called an anti-aging factor. This experiment measured the effect of the peptide of the present invention on the hyaluronic acid content to determine whether the peptide of the invention can increase the hyaluronic acid content and thus play a role in moisturizing and anti-aging repair.
The results of the effects of the test samples on the hyaluronic acid content are shown in FIG. 9. The results showed that, compared with the blank control group, the polypeptide groups could increase the hyaluronic acid content to different degrees, with the peptide (4) and peptide (20) of the present invention showing more superior technical effects.
It can be seen from this that the peptide of the present invention can increase the content of hyaluronic acid, thereby enhancing the skin barrier, replenishing moisture on the skin surface, making the skin surface moisturized and smooth, and thus exhibiting the effects of moisturizing and anti-aging repair.

EXAMPLE 11 Filaggrin expression experiment

11.1 Reagents and materials

Fetal bovine serum, DMEM culture medium, PBS, and filaggrin (FLG) detection kit.

11.2 Instruments

A fluorescence microscope, a $CO_2$ incubator, an ultra-clean workbench, and a thermostat.

11.3 Cell lines

Human keratinocytes (HaCaT) were purchased from the Kunming Cell Bank of China Center for Type Culture Collection under the Chinese Academy of Sciences.

11.4 Samples to be tested

Polypeptide group: Peptide (4), peptide (9), peptide (13) and peptide (20). The test concentrations of all the above samples were 10 ppm and 50 ppm.
Blank control group: PBS.
UV group: UV radiation with PBS.

11.5 Experimental protocol

The frozen HaCaT cells were cultured and passaged at a ratio of 1:2 to about the 5th generation, and the cells in better growth were selected as experiment subjects. The cell suspension was taken and inoculated into a 96-well plate at $10^4$ cells/well, complete culture medium was added, and the mixture was cultured in a 5% $CO_2$ incubator at 37°C for 24 h. A UV photoaging model was established when the cells reached approximately 80% confluence.

The original culture medium solution was sucked out using a vacuum pump, and grouped. In the blank control group, 200 μL of PBS was added, and supplemented with the culture medium to 1200 μL, while no UV irradiation was performed. For the UV group and the polypeptide groups, a proper amount of PBS was added to wash the culture repeatedly until colorless, followed by adding 200 μL of PBS and irradiating under an 80 mJ/cm$^2$ UV light. The distance between the light source and the culture bottle was 15 cm. After irradiation, the PBS was removed. In the UV group, a PBS solution and the culture medium were added to 1200 μL; in the polypeptide groups, the culture medium and the samples diluted in a certain ratio were added to 1200 μL. The blank control group, the UV group, and the polypeptide groups were further incubated in a 5% $CO_2$ incubator at 37°C for 24 h.

After incubation, the cells were digested, grown on a cell climbing slice for 12 hours and then fixed. The cells were stained using a filaggrin (FLG) detection kit and observed under a fluorescence microscope after staining.

11.6 Experimental results

Filaggrin (FLG) is an important molecule that links keratin fibers in the stratum corneum of human skin. It can prevent transepidermal water loss and invasion of external substances. Therefore, improving the expression of FLG can enhance or repair the skin barrier. In this experiment, 80 mJ/cm$^2$ UV energy was selected for radiation to establish a skin stratum corneum cell model, and the effect of the sample on FLG expression was detected to analyze whether the peptides of the present invention can promote FLG expression.

The effect results of the test samples on FLG expression are shown in FIG. 10. Compared with the blank control group, the FLG content in the UV group was significantly decreased after UV radiation (as shown in the light-colored part of FIG. 10), indicating that the experimental cell model was successfully established. Compared with the UV group, the polypeptide groups all increased the FLG content to different degrees in the range of 10-50 ppm and promoted FLG expression, demonstrating an excellent effect in promoting FLG expression. Among them, peptide (13) achieved the optimum technical effect.

It can be seen from this that the peptide of the present invention can promote the expression of FLG and enhance or repair the skin barrier.

EXAMPLE 12 Hyaluronidase inhibition experiment

12.1 Experimental instruments and reagents

Spectrophotometer, phosphate buffered saline (PBS), hyaluronic acid, and hyaluronidase.

12.2 Samples to be tested

Peptide (13) and peptide (14). The test concentrations of all the above samples were 50 ppm and 100 ppm.

12.3 Experimental protocol

The experiment was divided into four groups: blank control group A1 (PBS + hyaluronidase), enzyme-free blank control group A2 (PBS + solvent without hyaluronidase), sample group B1 (samples to be tested + hyaluronidase), and enzyme-free sample group B2 (samples to be tested + solvent without hyaluronidase). The total reaction volume of each group was the same.

According to the grouping, 100 μL of $CaCl_2$ (2.5 mmol/L) was mixed with 500 μL of hyaluronidase (500 U/mL) or solvent without hyaluronidase, and 20 μL of the mixture was taken and shaken in a 37°C constant temperature gas bath oscillator for 20 min. The blank control group was added with 20 μL of PBS and shaken in a 37°C constant temperature gas bath oscillator for 20 min. The sample groups were added with 20 μL of samples at different concentrations and shaken in a 37°C constant temperature gas bath oscillator for 20 min. The above groups were added with 40 μL of hyaluronic acid and shaken in a 37°C constant temperature gas bath oscillator for 40 min. 10 μL of acetylacetone solution was added and shaken in a 50°C constant temperature gas bath oscillator for 10 min, then placed in an oven at 75°C for 40 min and at 50°C for 10 min. After the reaction was completed and cooled, the plastic wrap was quickly removed to avoid the generation of water vapor. Then 100 μL of 96% anhydrous ethanol and 10 μL of Ehrlich reagent were added, and the absorbances were

measured at 492 nm.

$$\text{Hyaluronidase inhibition rate (\%)} = \frac{(A1-A2)-(B1-B2)}{A1-A2} \times 100\%.$$

12.4 Experimental results

Hyaluronidase can decompose hyaluronic acid in the body, converting it into low-molecular-weight acidic irritant, thereby causing the release of histamine and inducing sensitive symptoms in the body. In addition, the reduction of hyaluronic acid will destroy the skin barrier function, leading to the loss of moisture inside the skin. Therefore, inhibiting the activity of hyaluronidase can achieve the effects of soothing, moisturizing and repairing the skin barrier.

The effect results of test samples on inhibition rate of hyaluronidase are shown in FIG. 11. The results showed that both peptide (13) and peptide (14) inhibited the activity of hyaluronidase at the tested concentrations. At 100 ppm, the hyaluronidase inhibition rate of peptide (13) was 49.40%, and the hyaluronidase inhibition rate of peptide (14) was 40.96%.

The difference between peptide (13) and peptide (14) is that the C-terminus of the peptide chain of peptide (13) is in the form of amidation (-$NH_2$), while the C-terminus of the peptide chain of peptide (14) is in the form of -OH. Experiments show that the peptides of the present invention, regardless of whether the C-terminus of the peptide chain is -$NH_2$ or -OH, have the same efficacy in inhibiting the activity of hyaluronidase, and play roles in soothing, moisturizing and repairing the skin barrier.

In summary, the peptide of the present invention can improve cell activity, promote cell proliferation and migration, enhance or repair the skin barrier, and has the effect of anti-aging repair. It also has the effect of anti-aging or photoaging; and soothing and moisturizing.

EXAMPLE 13 Preparation of liposomes containing peptide (4)

| Ingredients | by weight % |
|---|---|
| Dipalmitoyl phosphatidylcholine | 4.0 |
| Peptide (4) | 0.2 |
| Preservative | 0.5 |
| Water | Balance to 100 |

Preparation method: Dipalmitoyl phosphatidylcholine was weighed and dissolved in chloroform. Solvent was evaporated under vacuum until a thin layer of phospholipid was obtained. This layer was treated with an aqueous solution of peptide with the required concentration at 55°C for hydration to obtain multilamellar liposomes. The multilamellar liposomes were subjected to high pressure homogenization treatment to obtain smaller and uniform unilamellar liposomes.

EXAMPLE 14 Preparation of an essence containing the peptide (9)

| Ingredients | by weight % |
|---|---|
| Sodium hyaluronate | 0.05 |
| Butylene glycol | 3.00 |
| Xanthan gum | 0.10 |
| 1,2-Pentanediol | 2.00 |
| 1,2-Hexanediol | 0.50 |
| Trehalose | 2.00 |
| Peptide (9) | 0.03 |
| Purified water | Balance to 100 |

Preparation method: The purified water was heated to 85°C with stirring, and kept at this temperature for 30 min.

The sodium hyaluronate and xanthan gum were pre-dissolved in butylene glycol, then added to the water and stirred for complete dissolution. The mixture was stirred and cooled to 35°C, and the remaining ingredients were added, and stirred uniformly to obtain the essence.

EXAMPLE 15 Preparation of an emulsion containing the peptide (13)

| Ingredients | by weight % |
|---|---|
| A Cetearyl alcohol (and) cetearyl glucoside | 5.0 |
| Jojoba oil | 5.0 |
| Mineral oil | 5.0 |
| Isopropyl palmitate | 7.0 |
| B Glycerin | 5.0 |
| Allantoin | 0.1 |
| Polyacrylamide (and) C13-14 isoparaffin (and) laureth-7 | 0.3 |
| Water | Balance to 100 |
| C Preservative | 0.5 |
| D Perfume | 0.5 |
| Peptide (13) | 0.01 |

Method of preparation: the peptide (13) was dissolved in water to obtain a polypeptide solution; cetearyl alcohol (and) cetearyl glucoside, jojoba oil, mineral oil, and isopropyl palmitate were heated to 85°C, and stirred evenly to afford phase A; glycerin, allantoin, polyacrylamide (and) C13-14 isoparaffin (and) laureth-7 were dissolved in water, and heated to 85°C to afford phase B; phase A was quickly added into phase B, homogenized at a constant temperature for 3-5 min and then cooled; when the above mixture was cooled to below 60°C, a preservative was added and stirred evenly. After cooling to below 45°C, the polypeptide solution and perfume were added to afford an emulsion.

EXAMPLE 16 A microemulsion composition containing the peptide (14)

| Ingredients | by weight % |
|---|---|
| A Sodium diethylhexyl sulfosuccinate | 1.35 |
| Isostearic acid | 7.65 |
| B Water | 0.2 |
| Denatured alcohol | 0.8 |
| C Ethylhexyl cocoate | Balance to 100 |
| D Peptide (14) | 0.005 |

Preparation method: The ingredients of B phase were weighed according to dosage above, and added to a container. Next, the D phase was added to the B phase, and homogenized under continuous stirring. Subsequently, the A phase was added to the mixture. Finally, the C phase was added, and stirred uniformly to obtain the microemulsion composition.

EXAMPLE 17 Preparation of a skin toner containing the peptide (20)

| Ingredients | by weight % |
|---|---|
| Propylene glycol | 1.0 |
| Allantoin | 0.3 |

(continued)

| Ingredients | by weight % |
|---|---|
| Glycerin | 1.0 |
| PEG-7 glyceryl cocoate | 1.0 |
| Peptide (20) | 0.005 |
| Preservative | 0.5 |
| Perfume | 0.5 |
| Water | Balance to 100 |

Preparation method: Allantoin and glycerin were dissolved in water and heated to 85°C at which the temperature was kept for 30 min. PEG-7 glyceryl cocoate and the peptide (20) were dissolved in water; the above solutions were mixed after cooling, and stirred uniformly to afford a mixed solution. Propylene glycol, a preservative, and perfume were added to the above mixed solution in turn, and water was added with stirring evenly to afford the skin toner.

Although preferred embodiments of the present invention have been described, those skilled in the art can make additional changes and modifications to these embodiments once they have knowledge of the basic creative concept. Therefore, the attached claims are intended to be interpreted as including the preferred embodiments and all the changes and modifications falling within the scope of the embodiments of the present invention.

Finally, it should be noted that relational terms used herein such as first and second are only used to distinguish one entity or operation from another, and do not necessarily require or imply any actual relationship or order between these entities or operations. Moreover, the terms "include", "comprise", or any other variants thereof are intended to encompass non-exclusive inclusion, such that a process, method, item, or terminal device that includes a series of elements not only includes those elements, but also includes other elements not explicitly listed, or also includes elements inherent to such process, method, item, or terminal device. Without further limitations, the element defined by the statement "including one..." does not exclude the existence of other identical elements in the process, method, item, or terminal device that includes the element in question.

A peptide and a composition and use thereof provided according to the present invention are detailed in the above description. The principles and embodiments of this invention are illustrated by applying specific examples herein. The description of the above examples is only used to help understand the method and its core idea of the present invention. Meanwhile, changes may be made to the specific embodiments and application range for those skilled in the art based on the idea of the present application. In summary, the contents of this specification should not be construed as a limitation to the present application.

## Claims

1. A peptide represented by formula (I), or a stereoisomer thereof, or a mixture of stereoisomers thereof, or a salt thereof,

$$R_1\text{-}X_1\text{-}X_2\text{-}X_3\text{-Leu-Tyr-}X_4\text{-}X_5\text{-}R_2 \qquad (I)$$

in formula (I),

$X_1$ is selected from -Gly-, -Lys- or a bond;
$X_2$ is selected from -Nle-, -Val-, -Leu- or -Ile-;
$X_3$ is selected from -Lys-, -Arg- or -His-;
$X_4$ is selected from -Gly- or a bond;
$X_5$ is selected from -Ser- or a bond;
$R_1$ is selected from H or $R_3$-CO-, wherein $R_3$ is selected from substituted or unsubstituted alkyl, substituted or unsubstituted alkenyl;
$R_2$ is selected from -$NR_4R_5$ or -$OR_4$, wherein each of $R_4$ and $R_5$ is independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally selected from methyl, ethyl, isopropyl, isobutyl, tert-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon

atoms; optionally having 2-14 carbon atoms; optionally having 2-12 carbon atoms; optionally having 2, 3, 4, 5, or 6 carbon atoms); the alkenyl has one or more carbon-carbon double bonds, optionally has 1, 2 or 3 conjugated or non-conjugated carbon-carbon double bonds; the alkenyl is bonded to the rest portions of a molecule through a single bond; and is optionally selected from vinyl, oleyl, or linoleyl;
optionally, the substituent in the "substituted alkyl" or "substituted alkenyl" is selected from $C_1$-$C_4$ alkyl; hydroxyl; $C_1$-$C_4$ alkoxy; amino; $C_1$-$C_4$ aminoalkyl; $C_1$-$C_4$ carbonyloxy; $C_1$-$C_4$ oxycarbonyl; halogen (such as fluorine, chlorine, bromine, and iodine); cyano; nitro; azide; $C_1$-$C_4$ alkylsulfonyl; thiol; $C_1$-$C_4$ alkylthio; $C_6$-$C_{30}$ aryloxy such as phenoxy; $-NR_b(C{=}NR_b)NR_bR_c$, wherein $R_b$ and $R_c$ are independently selected from H, $C_1$-$C_4$ alkyl, $C_2$-$C_4$ alkenyl, $C_2$-$C_4$ alkynyl, $C_3$-$C_{10}$ cycloalkyl, $C_6$-$C_{18}$ aryl, $C_7$-$C_{17}$ aralkyl, a heterocyclic group having 3 to 10 members, or an amino protecting group.

2. The peptide represented by formula (I) according to claim 1, or a stereoisomer thereof, or a mixture of stereoisomers thereof, or a salt thereof, **characterized in that** $R_1$ is selected from H, acetyl, tert-butyryl, hexanoyl, 2-methylhexanoyl, octanoyl, decanoyl, lauroyl, myristoyl, palmitoyl, stearoyl, oleoyl or linoleoyl; $R_4$ and $R_5$ are independently selected from H, methyl, ethyl, hexyl, dodecyl or hexadecyl;

optionally, $R_1$ is selected from H, acetyl, lauroyl, myristoyl or palmitoyl; $R_4$ is H and $R_5$ is selected from H, methyl, ethyl, hexyl, dodecyl or hexadecyl;
optionally, $R_1$ is H, acetyl or palmitoyl; $R_2$ is -OH or $-NH_2$.

3. The peptide represented by formula (I) according to claim 1, or a stereoisomer thereof, or a mixture of stereoisomers thereof, or a salt thereof, **characterized in that** the peptide is selected from the following peptides (1)-(40):

(1) H-Gly-Nle-Lys-Leu-Tyr-OH;
(2) Ac-Gly-Nle-Lys-Leu-Tyr-$NH_2$;
(3) Palm-Gly-Nle-Lys-Leu-Tyr-OH;
(4) Palm-Gly-Nle-Lys-Leu-Tyr-$NH_2$;
(5) H-Val-Lys-Leu-Tyr-Gly-Ser-OH;
(6) H-Val-Lys-Leu-Tyr-Gly-Ser-$NH_2$;
(7) Ac-Val-Lys-Leu-Tyr-Gly-Ser-$NH_2$;
(8) Palm-Val-Lys-Leu-Tyr-Gly-Ser-OH;
(9) Palm-Val-Lys-Leu-Tyr-Gly-Ser-$NH_2$;
(10) H-Lys-Val-Lys-Leu-Tyr-OH;
(11) H-Lys-Val-Lys-Leu-Tyr-$NH_2$;
(12) Ac-Lys-Val-Lys-Leu-Tyr-$NH_2$;
(13) Palm-Lys-Val-Lys-Leu-Tyr-$NH_2$;
(14) Palm-Lys-Val-Lys-Leu-Tyr-OH;
(15) H-Gly-Nle-Arg-Leu-Tyr-OH;
(16) H-Gly-Nle-Arg-Leu-Tyr-$NH_2$;
(17) Ac-Gly-Nle-Arg-Leu-Tyr-OH;
(18) Ac-Gly-Nle-Arg-Leu-Tyr-$NH_2$;
(19) Palm-Gly-Nle-Arg-Leu-Tyr-OH;
(20) Palm-Gly-Nle-Arg-Leu-Tyr-$NH_2$;
(21) H-Lys-Leu-Lys-Leu-Tyr-OH;
(22) Ac-Lys-Leu-Lys-Leu-Tyr-$NH_2$;
(23) Ac-Gly-Ile-Lys-Leu-Tyr-$NH_2$;
(24) H-Gly-Leu-Arg-Leu-Tyr-Ser-OH;
(25) H-Gly-Val-Lys-Leu-Tyr-$NH_2$;
(26) Palm-Gly-Val-His-Leu-Tyr-$NH_2$;
(27) Ac-Gly-Nle-Arg-Leu-Tyr-Gly-OH;
(28) H-Nle-Lys-Leu-Tyr-Gly-OH;
(29) Ac-Ile-Lys-Leu-Tyr-Gly-Ser-$NH_2$;
(30) H-Lys-Nle-Arg-Leu-Tyr-OH;
(31) H-Ile-Lys-Leu-Tyr-Ser-$NH_2$;
(32) Ac-Gly-Nle-Lys-Leu-Tyr-OH;
(33) Palm-Gly-Leu-Lys-Leu-Tyr-$NH_2$;
(34) H-Gly-Leu-Lys-Leu-Tyr-Gly-OH;
(35) Ac-Ile-Lys-Leu-Tyr-Gly-$NH_2$;

(36) H-Ile-Arg-Leu-Tyr-Gly-Ser-OH;
(37) H-Val-His-Leu-Tyr-Gly-$NH_2$;
(38) Ac-Val-His-Leu-Tyr-Gly-Ser-OH;
(39) Palm-Gly-Leu-Arg-Leu-Tyr-Gly-$NH_2$;
(40) H-Gly-Leu-Arg-Leu-Tyr-Ser-OH.

**4.** The peptide represented by formula (I) according to claim 3, or a stereoisomer thereof, or a mixture of stereoisomers thereof, or a salt thereof, **characterized in that** it is selected from the following peptide (3), peptide (4), peptide (8), peptide (9), peptide (13), peptide (14), peptide (19), and peptide (20), specifically,

(3) Palm-Gly-Nle-Lys-Leu-Tyr-OH;
(4) Palm-Gly-Nle-Lys-Leu-Tyr-$NH_2$;
(8) Palm-Val-Lys-Leu-Tyr-Gly-Ser-OH;
(9) Palm-Val-Lys-Leu-Tyr-Gly-Ser-$NH_2$;
(13) Palm-Lys-Val-Lys-Leu-Tyr-$NH_2$;
(14) Palm-Lys-Val-Lys-Leu-Tyr-OH;
(19) Palm-Gly-Nle-Arg-Leu-Tyr-OH;
(20) Palm-Gly-Nle-Arg-Leu-Tyr-$NH_2$.

**5.** The peptide represented by formula (I) according to any of claims 1-4, or a stereoisomer thereof, or a mixture of stereoisomers thereof, or a salt thereof, **characterized in that**

the salt includes a metal salt of the peptide represented by formula (I), wherein the metal includes lithium, sodium, potassium, calcium, magnesium, manganese, copper, zinc or aluminum;
optionally, the salt includes a salt formed from the peptide represented by formula (I) and an organic base, wherein the organic base includes ethylamine, diethylamine, ethylenediamine, ethanolamine, diethanolamine, arginine, lysine, histidine or piperazine;
optionally, the salt includes a salt formed from the peptide represented by formula (I) and an inorganic acid or an organic acid, wherein the organic acid includes acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid or gluconic acid; the inorganic acid includes hydrochloric acid, sulfuric acid, boric acid or carbonic acid.

**6.** A composition, **characterized in that** it includes an effective amount of the peptide represented by formula (I) according to any of claims 1-5, or a stereoisomer thereof, or a mixture of stereoisomers thereof, or a salt thereof, and at least one excipient and optional an adjuvant;
optionally, the adjuvant is selected from analgesics, agents inhibiting PAR-2 activity, collagen synthesis stimulating agents, agents modulating PGC-1 $\alpha$ synthesis, agents modulating PPAR $\gamma$ activity, agents increasing or reducing the triglyceride content of adipocytes, agents stimulating or delaying adipocyte differentiation, lipolytic agents or agents stimulating lipolysis, anti-cellulite agents, adipogenic agents, inhibitors of acetylcholine-receptor aggregation, agents inhibiting muscle contraction, anticholinergic agents, elastase inhibiting agents, matrix metalloproteinase inhibiting agents, melanin synthesis stimulating or inhibiting agents, whitening or depigmenting agents, propigmenting agents, self-tanning agents, antiaging agents, NO-synthase inhibiting agents, 5 $\alpha$ -reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, substances that retain moisture, alpha hydroxy acids, beta hydroxy acids, moisturizers, hydrolytic epidermal enzymes, vitamins, amino acids, proteins, pigments, dyes, biopolymers, gelling polymers, thickening agents, surfactants, softening agents, binding agents, preservatives, anti-wrinkle agents, agents able to reduce or treat the bags under the eyes, keratolytic agents, antimicrobial agents, agents stimulating the synthesis of dermal or epidermal macromolecules and/or capable of inhibiting or preventing their degradation, elastin synthesis-stimulation agents, decorin synthesis-stimulation agents, laminin synthesis-stimulation agents, defensin synthesis-stimulating agents, chaperone synthesis-stimulating agents, cAMP synthesis-stimulating agents, hyaluronic acid synthesis-stimulating agents, fibronectin synthesis-stimulating agents, sirtuin synthesis-stimulating agents, agents stimulating the synthesis of lipids and components of the stratum corneum, ceramides, fatty acids, agents that inhibit collagen degradation, agents that inhibit elastin degradation, agents that inhibit serine proteases, agents stimulating fibroblast proliferation, agents stimulating keratinocyte proliferation, agents stimulating adipocyte proliferation, agents stimulating melanocyte proliferation, agents stimulating keratinocyte differentiation, agents that inhibit acetylcholinesterase, skin relaxant agents, glycosaminoglycan synthesis-stimulating agents, antihyperkeratosis agents, comedolytic agents, anti-psoriasis agents, anti-eczema agents, DNA repair agents, DNA protecting agents, stabilizers, anti-itching agents, agents for the treatment and/or care of sensitive skin, firming agents, redensifying agents, restructuring agents, anti-stretch mark agents, agents regulating sebum production, antiperspirant agents, agents stimulating healing, coadjuvant healing agents, agents stimulating reepithelization, coadjuvant re-epithelization agents, cytokines, calming agents, anti-inflammatory agents, anesthetic agents, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, perfumes, chelating agents, plant extracts, essential oils, marine extracts, agents obtained from a biofermentation process, mineral salts, cell extracts, sunscreens, and organic or mineral photoprotective agents active against ultraviolet A and/or B rays or mixtures thereof.

**7.** The composition according to claim 6, **characterized in that** a preparation of the composition is selected from creams, oils, balms, foams, lotions, gels, liniments, sera, ointments, mousses, powders, bars, pencils, sprays, aerosols, capsules, tablets, granules, solutions, suspensions, emulsions, elixirs, polysaccharide films or jellies.

**8.** A delivery system or sustained release system, **characterized in that** it comprises an effective amount of the peptide represented by formula (I) according to any of claims 1-5, or a stereoisomer thereof, or a mixture of stereoisomers thereof, or a salt thereof, or the composition according to claim 6 or 7;
the delivery system or sustained release system is selected from liposomes, oleosomes, non-ionic surfactant liposome vesicles, ethosomes, millicapsules, microcapsules, nanocapsules, nanostructured lipid carriers, sponges, lipoid vesicles, micelles, millispheres, microspheres, nanospheres, lipospheres, microemulsions, nanoemulsions, milliparticles, microparticles or nanoparticles.

**9.** Use of the peptide represented by formula (I) according to any of claims 1-5, or a stereoisomer thereof, or a mixture of stereoisomers thereof, or a salt thereof, or the composition according to claim 6 or 7, or the delivery system or sustained release system according to claim 8 in the preparation of a composition for anti-aging or photoaging.

**10.** Use of the peptide represented by formula (I) according to any of claims 1-5, or a stereoisomer thereof, or a mixture of stereoisomers thereof, or a salt thereof, or the composition according to claim 6 or 7, or the delivery system or sustained release system according to claim 8 in the preparation of a composition for anti-aging repair, the anti-aging repair including one or more of improving cell activity, promoting cell proliferation and migration, enhancing or repairing the skin barrier.

**11.** Use of the peptide represented by formula (I) according to any of claims 1-5, or a stereoisomer thereof, or a mixture of stereoisomers thereof, or a salt thereof, or the composition according to claim 6 or 7, or the delivery system or sustained release system according to claim 8 in the preparation of a composition for soothing.

**12.** Use of the peptide represented by formula (I) according to any of claims 1-5, or a stereoisomer thereof, or a mixture of stereoisomers thereof, or a salt thereof, or the composition according to claim 6 or 7, or the delivery system or sustained release system according to claim 8 in the preparation of a composition for moisturizing.

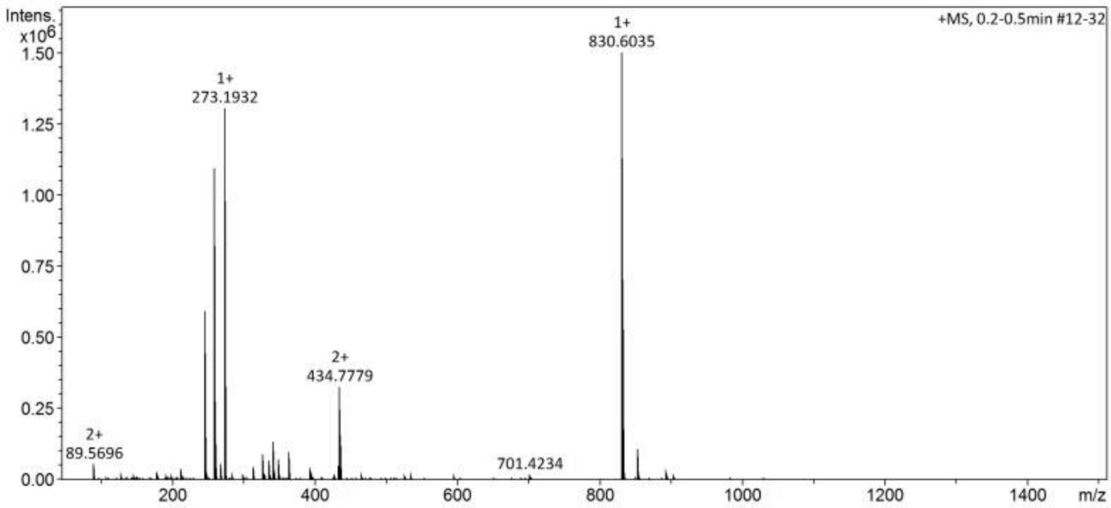


FIG. 1

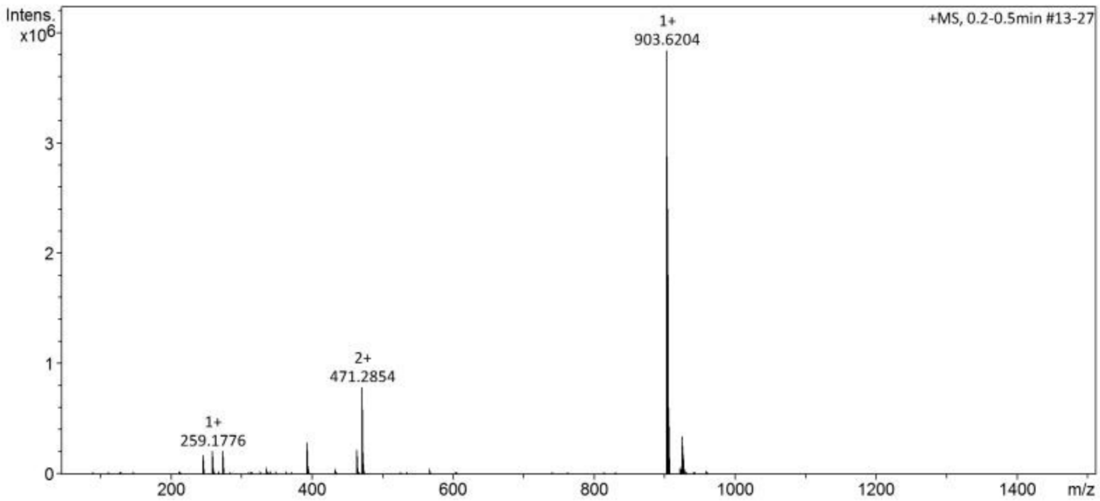


FIG. 2

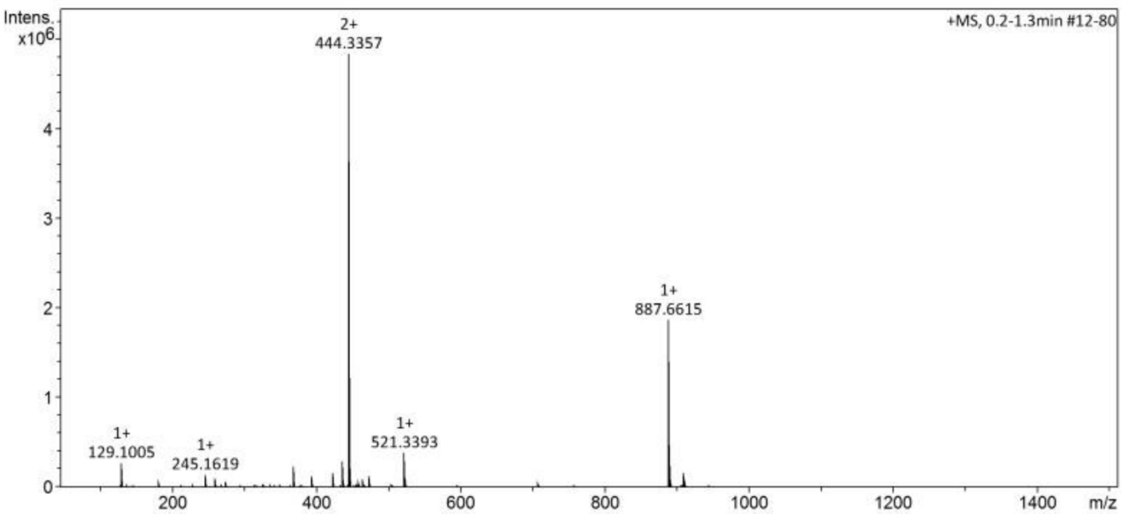


FIG. 3

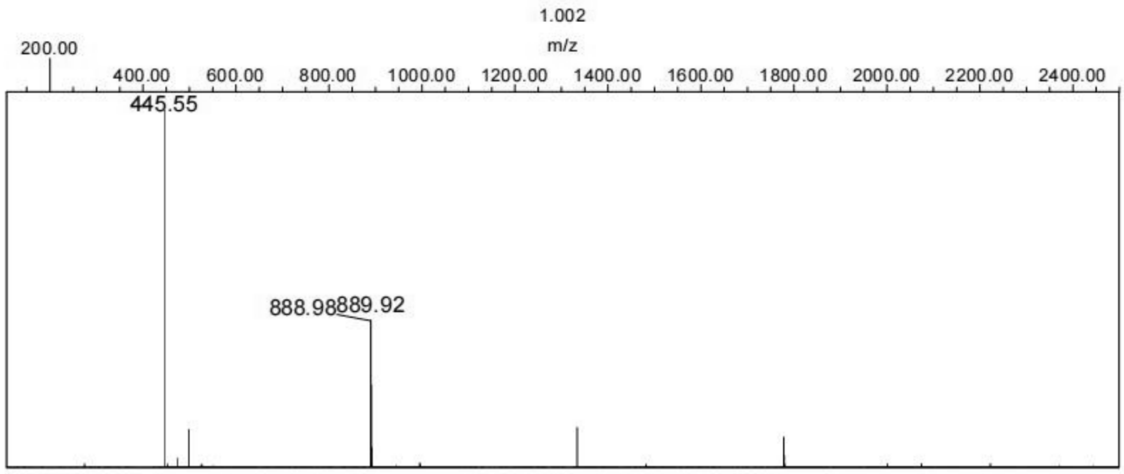
1.002
m/z
200.00
400.00
600.00
800.00
1000.00
1200.00
1400.00
1600.00
1800.00
2000.00
2200.00
2400.00
445.55
888.98
889.92

FIG. 4

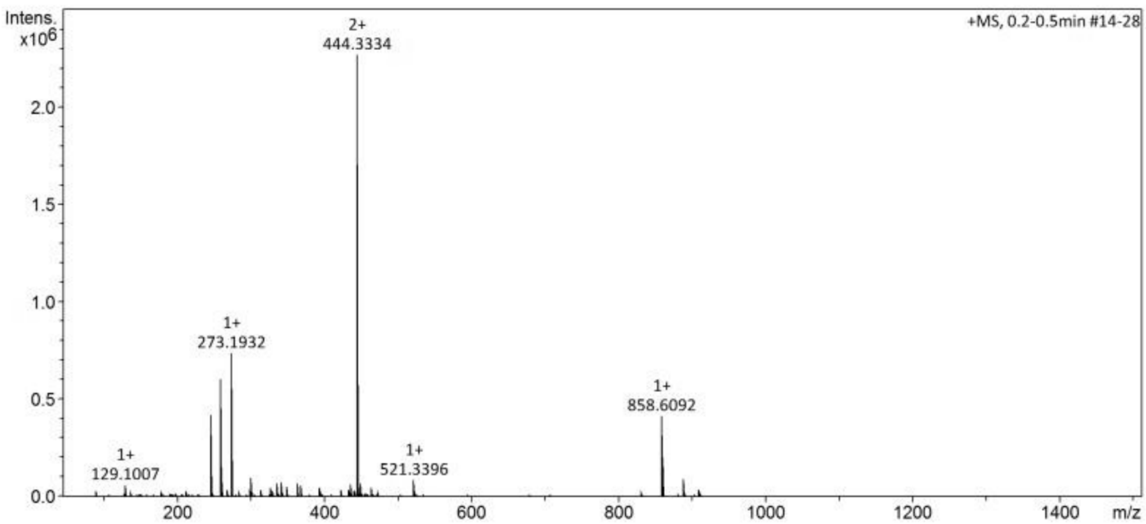
Intens.
x10^6
+MS, 0.2-0.5min #14-28
2+
444.3334
1+
273.1932
1+
858.6092
1+
129.1007
1+
521.3396
2.0
1.5
1.0
0.5
0.0
200
400
600
800
1000
1200
1400
m/z

FIG. 5

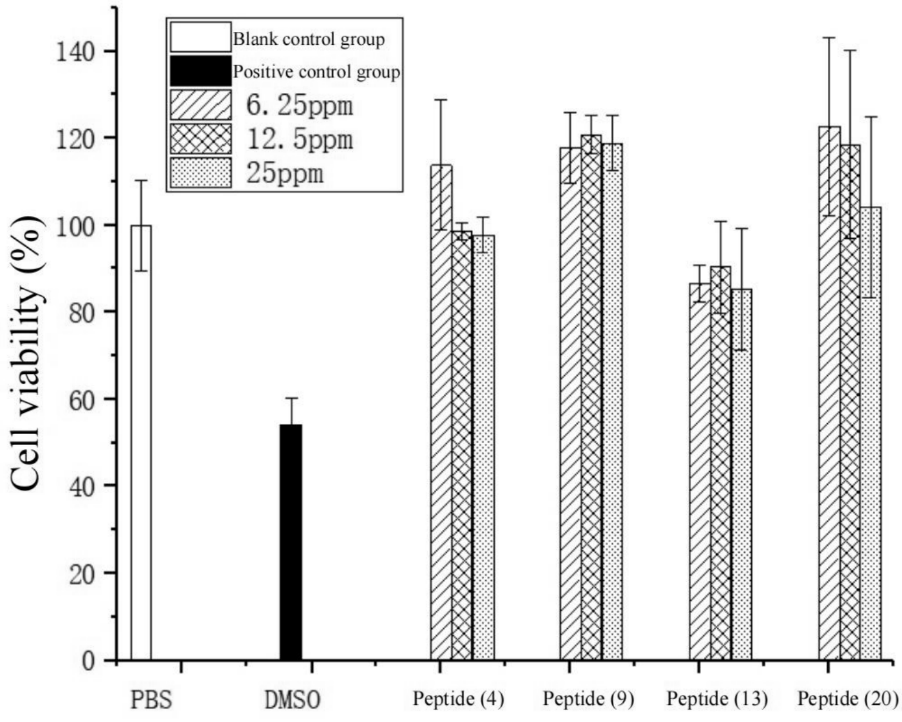

Blank control group
Positive control group
6.25ppm
12.5ppm
25ppm
Cell viability (%)
0
20
40
60
80
100
120
140
PBS
DMSO
Peptide (4)
Peptide (9)
Peptide (13)
Peptide (20)


FIG. 6

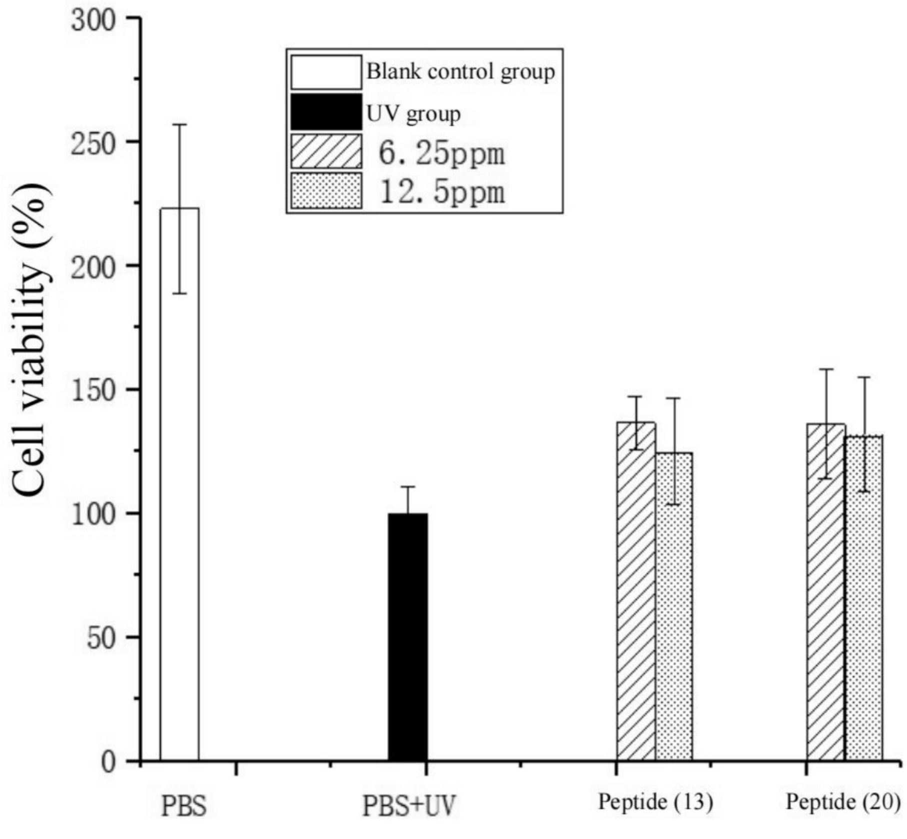

Blank control group
UV group
6.25ppm
12.5ppm
Cell viability (%)
0
50
100
150
200
250
300
PBS
PBS+UV
Peptide (13)
Peptide (20)


FIG. 7

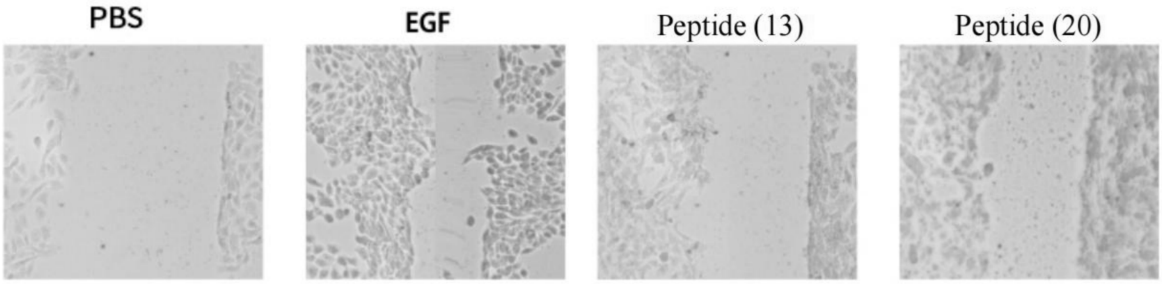
PBS
EGF
Peptide (13)
Peptide (20)

FIG. 8

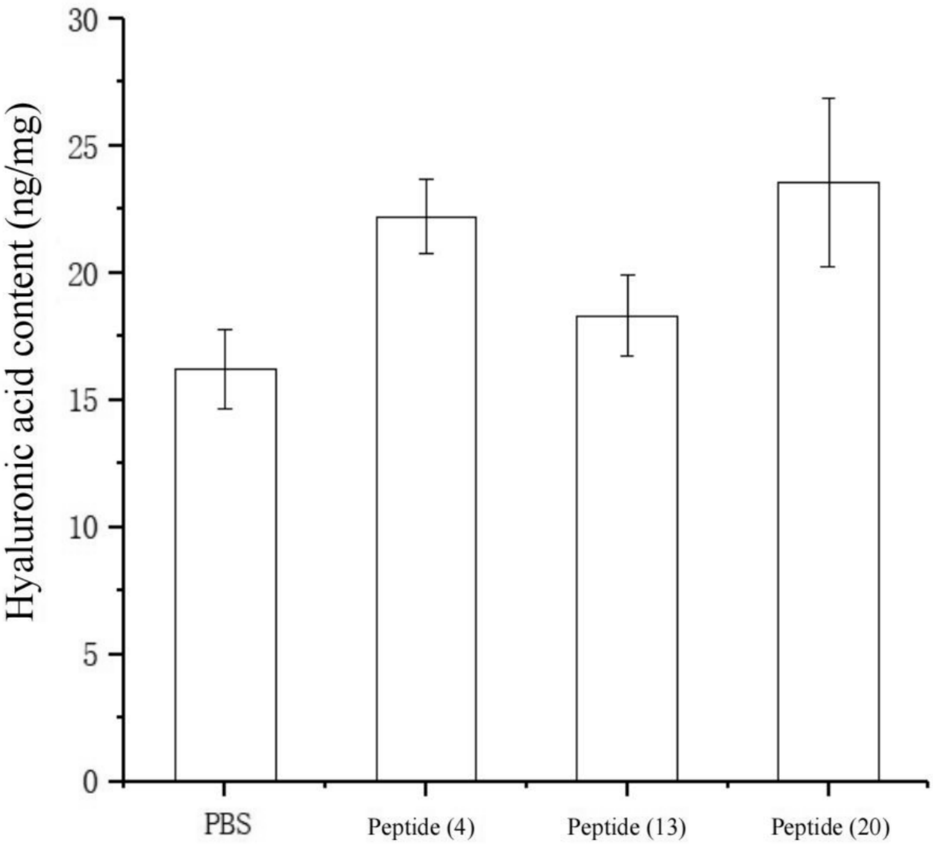
Hyaluronic acid content (ng/mg)
0
5
10
15
20
25
30
PBS
Peptide (4)
Peptide (13)
Peptide (20)

FIG. 9

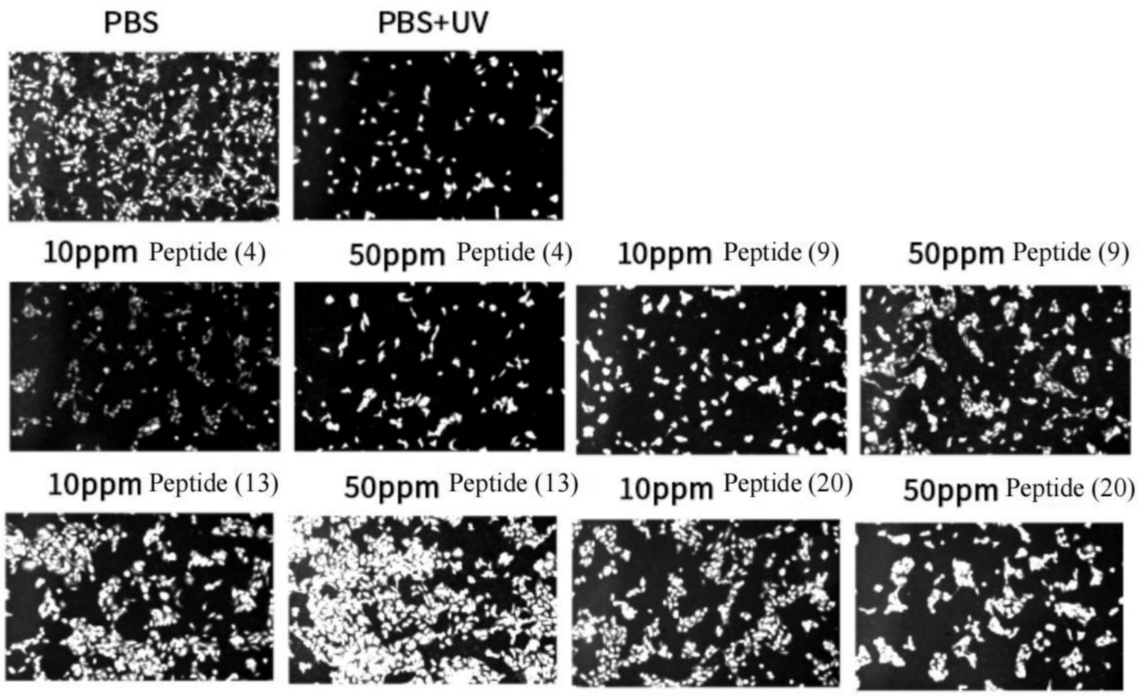
PBS
PBS+UV
10ppm Peptide (4)
50ppm Peptide (4)
10ppm Peptide (9)
50ppm Peptide (9)
10ppm Peptide (13)
50ppm Peptide (13)
10ppm Peptide (20)
50ppm Peptide (20)

FIG. 10

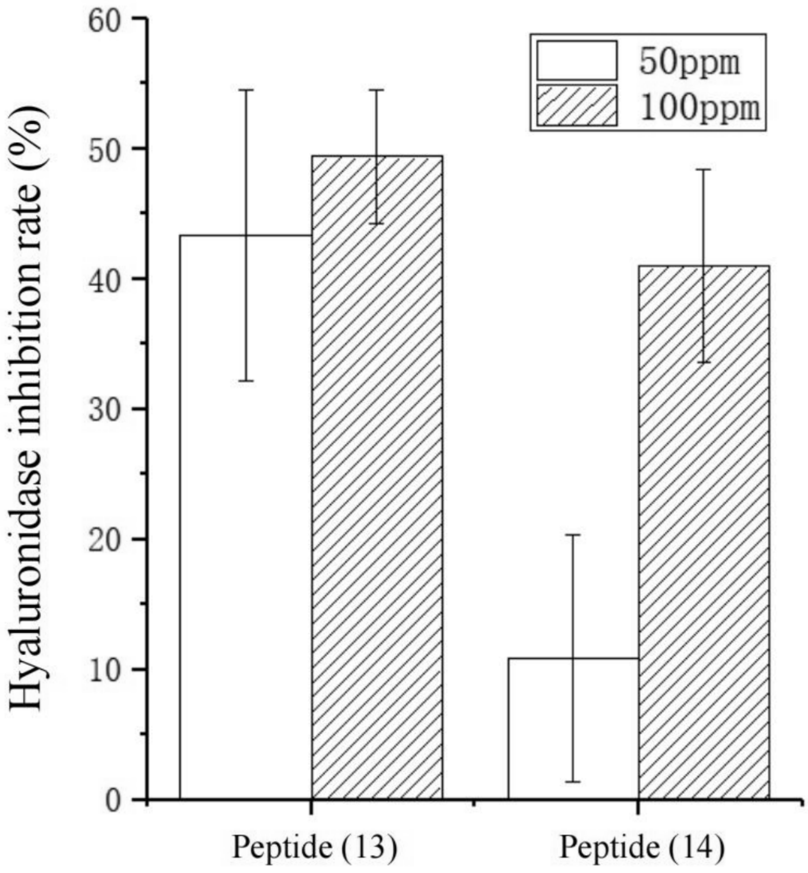
Hyaluronidase inhibition rate (%)
0
10
20
30
40
50
60
50ppm
100ppm
Peptide (13)
Peptide (14)

FIG. 11

**INTERNATIONAL SEARCH REPORT**

International application No.
**PCT/CN2024/088620**

**A. CLASSIFICATION OF SUBJECT MATTER**

C07K7/06(2006.01)i； A61K38/08(2019.01)i； A61P17/00(2006.01)i； A61P17/18(2006.01)i； A61P17/16(2006.01)i

According to International Patent Classification (IPC) or to both national classification and IPC

**B. FIELDS SEARCHED**

Minimum documentation searched (classification system followed by classification symbols)

IPC： C07K, A61K, A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, DPWI, VEN, CNKI, 万方数据, WANFANG DATA, Web of Science, 百度学术, Baidu Xueshu, HimmPat, incoPat, 读秀, DUXIU, 中国生物序列检索系统, Bio-Sequence Database of Chinese Patent, NCBI, EBI, STNext: 申请人, 发明人, Applicants, Inventors, 肽, 老化, 皱纹, 氧化, 透明质酸, peptide?, aging, wrinkl+, oxidation , hyaluronic acid, 肽1序列, sequence of peptide 1

**C. DOCUMENTS CONSIDERED TO BE RELEVANT**

| Category* | Citation of document, with indication, where appropriate, of the relevant passages | Relevant to claim No. |
|---|---|---|
| PX | CN 116284241 A (SHENZHEN WEIQI TECHNOLOGY CO., LTD.) 23 June 2023 (2023-06-23)<br>description, paragraphs 7-21, 24, 73-90 and 93-100 | 1-3, 5-12 |
| PX | CN 117327150 A (SHENZHEN WEIQI TECHNOLOGY CO., LTD.) 02 January 2024 (2024-01-02)<br>description, paragraphs 7-21, 24, 73-90 and 93-100 | 1-3, 5-12 |
| A | WO 2023015533 A1 (SHENZHEN WINKEY PHARMACEUTICAL R&D CO., LTD.) 16 February 2023 (2023-02-16)<br>claims 1-6, and description, page 13, paragraph 4 to page 14, paragraph 1, page 18, paragraphs 2-4, and page 22, paragraphs 2-5 | 1-3, 5-12 |
| A | WO 2023008913 A1 (SKINMED CO., LTD.) 02 February 2023 (2023-02-02)<br>claims 1-5 and 22-25, and description, paragraphs 30-43, 88-92 and 98-100, and sequences 600, 2945, 2967 and 2973 | 1-3, 5-12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 June 2024** | **04 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/088620**

**C. DOCUMENTS CONSIDERED TO BE RELEVANT**

| Category* | Citation of document, with indication, where appropriate, of the relevant passages | Relevant to claim No. |
| --- | --- | --- |
| A | CN 101076591 A (THE UNIVERSITY OF HONG KONG) 21 November 2007 (2007-11-21) sequence 2257 | 1-3, 5-12 |
| A | CN 115583988 A (SHENZHEN WINKEY PHARMACEUTICAL R&D CO., LTD.) 10 January 2023 (2023-01-10) claims 1-20 | 1-3, 5-12 |

Form PCT/ISA/210 (second sheet) (July 2022)

INTERNATIONAL SEARCH REPORT

International application No. **PCT/CN2024/088620**

| Box No. III | Observations where unity of invention is lacking (Continuation of item 3 of first sheet) |
|---|---|

This International Searching Authority found multiple inventions in this international application, as follows:

Invention 1: claims 1-3 and 5-12 (in part) relate to a peptide (1) or a stereoisomer thereof, or a mixture of stereoisomers thereof, or a salt thereof, a composition thereof, a delivery system or a sustained-release system, and the use thereof.

Invention 2: claims 1-3 and 5-12 (in part) relate to a peptide (2) or a stereoisomer thereof, or a mixture of stereoisomers thereof, or a salt thereof, a composition thereof, a delivery system or a sustained-release system, and the use thereof.

Invention 3: claims 1-12 (in part) relate to a peptide (3) or a stereoisomer thereof, or a mixture of stereoisomers thereof, or a salt thereof, a composition thereof, a delivery system or a sustained-release system, and the use thereof.

Invention 4: claims 1-12 (in part) relate to a peptide (4) or a stereoisomer thereof, or a mixture of stereoisomers thereof, or a salt thereof, a composition thereof, a delivery system or a sustained-release system, and the use thereof.

Invention 5: claims 1-3 and 5-12 (in part) relate to a peptide (5) or a stereoisomer thereof, or a mixture of stereoisomers thereof, or a salt thereof, a composition thereof, a delivery system or a sustained-release system, and the use thereof.

Invention 6: claims 1-3 and 5-12 (in part) relate to a peptide (6) or a stereoisomer thereof, or a mixture of stereoisomers thereof, or a salt thereof, a composition thereof, a delivery system or a sustained-release system, and the use thereof.

Invention 7: claims 1-3 and 5-12 (in part) relate to a peptide (7) or a stereoisomer thereof, or a mixture of stereoisomers thereof, or a salt thereof, a composition thereof, a delivery system or a sustained-release system, and the use thereof.

Invention 8: claims 1-12 (in part) relate to a peptide (8) or a stereoisomer thereof, or a mixture of stereoisomers thereof, or a salt thereof, a composition thereof, a delivery system or a sustained-release system, and the use thereof.

Invention 9: claims 1-12 (in part) relate to a peptide (9) or a stereoisomer thereof, or a mixture of stereoisomers thereof, or a salt thereof, a composition thereof, a delivery system or a sustained-release system, and the use thereof.

Invention 10: claims 1-3 and 5-12 (in part) relate to a peptide (10) or a stereoisomer thereof, or a mixture of stereoisomers thereof, or a salt thereof, a composition thereof, a delivery system or a sustained-release system, and the use thereof.

Invention 11: claims 1-3 and 5-12 (in part) relate to a peptide (11) or a stereoisomer thereof, or a mixture of stereoisomers thereof, or a salt thereof, a composition thereof, a delivery system or a sustained-release system, and the use thereof.

Invention 12: claims 1-3 and 5-12 (in part) relate to a peptide (12) or a stereoisomer thereof, or a mixture of stereoisomers thereof, or a salt thereof, a composition thereof, a delivery system or a sustained-release system, and the use thereof.

Invention 13: claims 1-12 (in part) relate to a peptide (13) or a stereoisomer thereof, or a mixture of stereoisomers thereof, or a salt thereof, a composition thereof, a delivery system or a sustained-release system, and the use thereof.

Invention 14: claims 1-12 (in part) relate to a peptide (14) or a stereoisomer thereof, or a mixture of stereoisomers thereof, or a salt thereof, a composition thereof, a delivery system or a sustained-release system, and the use thereof.

Invention 15: claims 1-3 and 5-12 (in part) relate to a peptide (15) or a stereoisomer thereof, or a mixture of stereoisomers thereof, or a salt thereof, a composition thereof, a delivery system or a sustained-release system, and the use thereof.

Invention 16: claims 1-3 and 5-12 (in part) relate to a peptide (16) or a stereoisomer thereof, or a mixture of stereoisomers thereof, or a salt thereof, a composition thereof, a delivery system or a sustained-release system, and the use thereof.

Invention 17: claims 1-3 and 5-12 (in part) relate to a peptide (17) or a stereoisomer thereof, or a mixture of stereoisomers thereof, or a salt thereof, a composition thereof, a delivery system or a sustained-release system, and the use thereof.

INTERNATIONAL SEARCH REPORT

International application No. PCT/CN2024/088620

**Box No. III Observations where unity of invention is lacking (Continuation of item 3 of first sheet)**

Invention 18: claims 1-3 and 5-12 (in part) relate to a peptide (18) or a stereoisomer thereof, or a mixture of stereoisomers thereof, or a salt thereof, a composition thereof, a delivery system or a sustained-release system, and the use thereof.

Invention 19: claims 1-12 (in part) relate to a peptide (19) or a stereoisomer thereof, or a mixture of stereoisomers thereof, or a salt thereof, a composition thereof, a delivery system or a sustained-release system, and the use thereof.

Invention 20: claims 1-12 (in part) relate to a peptide (20) or a stereoisomer thereof, or a mixture of stereoisomers thereof, or a salt thereof, a composition thereof, a delivery system or a sustained-release system, and the use thereof.

Invention 21: claims 1-3 and 5-12 (in part) relate to a peptide (21) or a stereoisomer thereof, or a mixture of stereoisomers thereof, or a salt thereof, a composition thereof, a delivery system or a sustained-release system, and the use thereof.

Invention 22: claims 1-3 and 5-12 (in part) relate to a peptide (22) or a stereoisomer thereof, or a mixture of stereoisomers thereof, or a salt thereof, a composition thereof, a delivery system or a sustained-release system, and the use thereof.

Invention 23: claims 1-3 and 5-12 (in part) relate to a peptide (23) or a stereoisomer thereof, or a mixture of stereoisomers thereof, or a salt thereof, a composition thereof, a delivery system or a sustained-release system, and the use thereof.

Invention 24: claims 1-3 and 5-12 (in part) relate to a peptide (24) or a stereoisomer thereof, or a mixture of stereoisomers thereof, or a salt thereof, a composition thereof, a delivery system or a sustained-release system, and the use thereof.

Invention 25: claims 1-3 and 5-12 (in part) relate to a peptide (25) or a stereoisomer thereof, or a mixture of stereoisomers thereof, or a salt thereof, a composition thereof, a delivery system or a sustained-release system, and the use thereof.

Invention 26: claims 1-3 and 5-12 (in part) relate to a peptide (26) or a stereoisomer thereof, or a mixture of stereoisomers thereof, or a salt thereof, a composition thereof, a delivery system or a sustained-release system, and the use thereof.

Invention 27: claims 1-3 and 5-12 (in part) relate to a peptide (27) or a stereoisomer thereof, or a mixture of stereoisomers thereof, or a salt thereof, a composition thereof, a delivery system or a sustained-release system, and the use thereof.

Invention 28: claims 1-3 and 5-12 (in part) relate to a peptide (28) or a stereoisomer thereof, or a mixture of stereoisomers thereof, or a salt thereof, a composition thereof, a delivery system or a sustained-release system, and the use thereof.

Invention 29: claims 1-3 and 5-12 (in part) relate to a peptide (29) or a stereoisomer thereof, or a mixture of stereoisomers thereof, or a salt thereof, a composition thereof, a delivery system or a sustained-release system, and the use thereof.

Invention 30: claims 1-3 and 5-12 (in part) relate to a peptide (30) or a stereoisomer thereof, or a mixture of stereoisomers thereof, or a salt thereof, a composition thereof, a delivery system or a sustained-release system, and the use thereof.

Invention 31: claims 1-3 and 5-12 (in part) relate to a peptide (31) or a stereoisomer thereof, or a mixture of stereoisomers thereof, or a salt thereof, a composition thereof, a delivery system or a sustained-release system, and the use thereof.

Invention 32: claims 1-3 and 5-12 (in part) relate to a peptide (32) or a stereoisomer thereof, or a mixture of stereoisomers thereof, or a salt thereof, a composition thereof, a delivery system or a sustained-release system, and the use thereof.

Invention 33: claims 1-3 and 5-12 (in part) relate to a peptide (33) or a stereoisomer thereof, or a mixture of stereoisomers thereof, or a salt thereof, a composition thereof, a delivery system or a sustained-release system, and the use thereof.

Invention 34: claims 1-3 and 5-12 (in part) relate to a peptide (34) or a stereoisomer thereof, or a mixture of stereoisomers thereof, or a salt thereof, a composition thereof, a delivery system or a sustained-release system, and the use thereof.

INTERNATIONAL SEARCH REPORT

International application No.
PCT/CN2024/088620

**Box No. III Observations where unity of invention is lacking (Continuation of item 3 of first sheet)**

Invention 35: claims 1-3 and 5-12 (in part) relate to a peptide (35) or a stereoisomer thereof, or a mixture of stereoisomers thereof, or a salt thereof, a composition thereof, a delivery system or a sustained-release system, and the use thereof.

Invention 36: claims 1-3 and 5-12 (in part) relate to a peptide (36) or a stereoisomer thereof, or a mixture of stereoisomers thereof, or a salt thereof, a composition thereof, a delivery system or a sustained-release system, and the use thereof.

Invention 37: claims 1-3 and 5-12 (in part) relate to a peptide (37) or a stereoisomer thereof, or a mixture of stereoisomers thereof, or a salt thereof, a composition thereof, a delivery system or a sustained-release system, and the use thereof.

Invention 38: claims 1-3 and 5-12 (in part) relate to a peptide (38) or a stereoisomer thereof, or a mixture of stereoisomers thereof, or a salt thereof, a composition thereof, a delivery system or a sustained-release system, and the use thereof.

Invention 39: claims 1-3 and 5-12 (in part) relate to a peptide (39) or a stereoisomer thereof, or a mixture of stereoisomers thereof, or a salt thereof, a composition thereof, a delivery system or a sustained-release system, and the use thereof.

Invention 40: claims 1-3 and 5-12 (in part) relate to a peptide (40) or a stereoisomer thereof, or a mixture of stereoisomers thereof, or a salt thereof, a composition thereof, a delivery system or a sustained-release system, and the use thereof.

The common technical feature comprised in inventions 1 to 40 is as follows: the polypeptide conforms to the structure shown in formula (I) $R_1$-$X_1$-$X_2$-$X_3$-Leu-Tyr-$X_4$-$X_5$-$R_2$. However, D1 (CN 101076591 A, 21 November 2007) discloses an isolated polypeptide (see sequence 2257), wherein the structure thereof is Val Lys Leu Tyr, which conforms to a preamble structure in formula (I), i.e. satisfies "$R_1$-$X_1$-$X_2$-$X_3$-Leu-Tyr-$X_4$-$X_5$"; moreover, the end of the polypeptide modified with groups such as -OH and -$NH_2$ is also the prior art, for example, D2 (WO 2023015533 A1, 16 February 2023) discloses (see claims 1-6) that chemical group $R_3$ is provided at the end of a polypeptide, which is selected from -$NR_5R_6$ or -$OR_5$, wherein each of $R_5$ and $R_6$ is independently selected from: H, a substituted or unsubstituted alkyl group, and a substituted or unsubstituted alkenyl group; and an example of a specific polypeptide modified with groups such as -OH and -$NH_2$ is provided. Therefore, the above-mentioned technical feature is not a special technical feature, and thus inventions 1-40 lack unity of invention (PCT Article 13(2)).

Form PCT/ISA/210 (continuation of first sheet) (July 2022)

**INTERNATIONAL SEARCH REPORT**

International application No. **PCT/CN2024/088620**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-3 and 5-12 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2024/088620**

| Patent document cited in search report | Publication date (day/month/year) | Patent family member(s) | Publication date (day/month/year) |
|---|---|---|---|
| CN 116284241 A | 23 June 2023 | None | |
| CN 117327150 A | 02 January 2024 | None | |
| WO 2023015533 A1 | 16 February 2023 | KR 20240042649 A | 02 April 2024 |
| WO 2023008913 A1 | 02 February 2023 | EP 4378950 A1 | 05 June 2024 |
| CN 101076591 A | 21 November 2007 | US 2009305282 A1 | 10 December 2009 |
| | | US 8092994 B2 | 10 January 2012 |
| | | US 2006018923 A1 | 26 January 2006 |
| | | US 7553944 B2 | 30 June 2009 |
| CN 115583988 A | 10 January 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310428893


### Non-patent literature cited in the description


- *Eur. J. Biochem.*, 1984, vol. 138, 9-37
- *J. Chem.*, 1989, vol. 264, 633-673